# EUROPEAN PATENT SPECIFICATION

(11) **EP 4 028 131 B1**
(45) Date of publication and mention of the grant of the patent: **22.11.2023**
(21) Application number: 20767475.5
(22) Date of filing: 14.08.2020
(51) Int. Cl.: A61P 31/14, A61K 31/5025, A61K 31/519, A61K 31/53, C07D 487/04

(54) **HETEROAROMATIC COMPOUNDS USEFUL IN THERAPY**
HETEROAROMATISCHE VERBINDUNGEN, DIE NÜTZLICH IN THERAPIEN SIND
COMPOSÉS HÉTÉROAROMATIQUES UTILES EN THÉRAPIE

(30) Priority: 20.08.2019 EP 19192642
(43) Date of publication of application: 20.07.2022
(73) Proprietor: CUROVIR AB, 391 27 Kalmar (SE)
(72) Inventor: WESTMAN, Jacob, 744 97 JÄRLÅSA (SE)
(74) Representative: Brann AB
(86) International application number: PCT/EP2020/072847
(87) International publication number: WO 2021/032611

(56) References cited:
- EP-A1- 3 079 693
- WO-A1-2013/123401

## Description

### FIELD OF THE INVENTION

The present invention relates generally to heteroaromatic compounds having usefulness in the treatment of conditions caused by certain viruses. More particularly, the invention relates to heteroaromatic compounds useful in the treatment of *Pneumoviridae* viral infections.

### BACKGROUND OF THE INVENTION

*Pneumoviridae* is a new virus family in the order Mononegavirales, derived from the paramyxoviral subfamily *Pneumovirinae.* Natural hosts include humans, cattle, and rodents. There are currently 5 species in the *Pneumoviridae* family, divided between 2 genera (Metapneumovirus and Orthopneumovirus). Pneumoviruses are pleomorphic, capable of producing spherical and filamentous enveloped virions (virus particles) that vary in size from 150 to 200 nm in diameter. The nucleocapsid consisting of a protein shell and viral nucleic acids has a helical symmetry. The genome is composed of negative-sense single-stranded RNA that is non-segmented. Viruses in this family are often associated with respiratory infections, and are transmitted through respiratory secretions. A common virus belonging to the *Pneumoviridae* family is the respiratory syncytial virus (RSV).

Respiratory syncytial virus (RSV) is one of the leading causes of lower respiratory tract infections in infants and young children. Over 95% of all children in the world have been infected with RSV by 2 years of age. RSV bronchiolitis is one of the leading causes of hospital admission of infants under 2 years of age worldwide and contributes greatly to the mortality rate in those infants. The impact of RSV infection is not limited to only young children. RSV infections can be dangerous for certain adults, especially those 65 years old or with chronic heart or lung disease or adults with weakened immune systems. Each year an estimated 177,000 older adults are hospitalized and 14,000 of them die in the United States due to RSV infection. RSV as well as rhinovirus are also described to be involved in COPD exacerbations.

Despite knowledge of the RSV viral genetics, structure, the transmission, and replication, there are still no antiviral therapies for RSV infections on the market. However, multiple antivirals are in development and being tested in clinical trials where all clinical tested compounds act on the virus and not on host targets.

Human Respiratory Syncytial Virus was first isolated in 1956 from a laboratory chimpanzee with a respiratory illness and was later discovered to be of human origin. RSV consists of two antigenic subtypes, A and B. Subtype B is characterized as the asymptomatic strains that the majority of individuals experience. The more severe illnesses, which usually predominate during outbreaks, are associated with subtype A strains.

The genome of RSV was completely sequenced in 1997. It is a linear single stranded negative-sense RNA consisting of 15,191 base pairs. The genome is found in the helical nucleocapsid. The genome encodes for 11 proteins including structural and non-structural. The virion of the RSV is enveloped with a lipid bilayer, which is obtained from the host's plasma membrane. It contains three surface glycoproteins, the attachment protein G, fusion protein F, and the small hydrophobic SH protein, which are separated from each other and can be seen as "spikes" that project out of the virion. The RSV F-protein is the most targeted protein in the different drug development pipelines (Xing et al 2019, Eur. J. Pediatric 178 (2), 131-138). There are however some few examples of so-called host-targeting antivirals under development such as the development of FASN inhibitors (Ohol et al, 2015, PLOS One, 10(12)1-20). Compounds suitable for the treatment of RSV are disclosed in WO2013/123401

The present inventor has previously developed compounds acting as PI4KIIIβ inhibitors. These compounds were shown to have an activity against various picornaviruses, cf. international applications Nos. PCT/EP2015/051177 (WO 2015/110491), PCT/EP2016/063383 (WO 2016/206999), PCT/EP2018/058522 (WO 2018/185120), and PCT/EP2019/072220 (WO 2020/074159). Picornaviruses are non-enveloped (naked) +ssRNA viruses belonging to the viral family *Picornaviridae,* a family of viruses in the order *Picornavirales.*

### SUMMARY OF THE INVENTION

The present invention is based on the unexpected finding that compounds, previously found to be active against picornaviruses, also show activity against an enveloped -ssRNA *Pneumoviridae* virus belonging to the order *Mononegavirales,* i.e. a very different virus family.

Consequently, a first aspect is compound of formula (I) 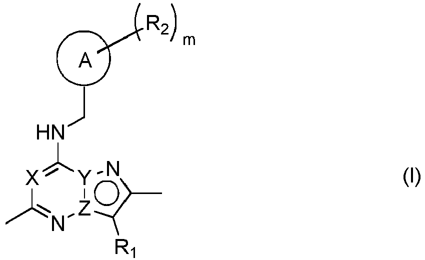 or a pharmaceutically acceptable salt thereof, wherein
X is CH, Y is C, and Z is N, or
X is CH, Y is N, and Z is C, or
X is N, Y is N, and Z is C;
R₁ is 3,4-dimethoxyphenyl or 1,3-dimethyl-1H-indazol-5-yl;
ring A is phenyl, or 5- or 6-membered heteroaryl;
m is 0, 1, 2 or 3;
each R₂ is independently selected from C1-C6 alkyl, halogen, R₃O, R₄R₅N, R₆R₇NS(O)₂, R₈R₉NC(O), R₁₀S(O)₂, R₁₁S(O)₂N(R₁₂), R₁₃C(O)O, R₁₄C(O)N(R₁₅), R₁₆OC(O), R₁₇C(O), R₁₈C(O)N(R₁₉)S(O)₂, R₂₀OC(O)N(R₂₁)S(O)₂, CN and -O⁻; and two R₂, attached to adjacent atoms of ring A, together with the atoms to which they are attached may form a 5- or 6-membered heterocyclic or carbocyclic ring;
each R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, and R₂₁ is independently selected from H and C1-C6 alkyl; and
any alkyl is optionally substituted by one or more F;
for use in the treatment of a *Pneumoviridae* viral infection.

A still further aspect is a pharmaceutical composition comprising a compound of formula (I), or a pharmaceutically acceptable salt thereof, and optionally a pharmaceutically acceptable excipient, for use in the treatment of a *Pneumoviridae* viral infection.

A further aspect is a novel compound of formula (I), which is 3-(3,4-dimethoxyphenyl)-2,6-dimethyl-N-(pyridin-4-ylmethyl)imidazo[1,2-b]pyridazin-8-amine, or a pharmaceutically acceptable salt thereof.

A still further aspect is 3-(3,4-dimethoxyphenyl)-2,6-dimethyl-N-(pyridin-4-ylmethyl)imidazo[1,2-b]pyridazin-8-amine, or a pharmaceutically acceptable salt thereof, for use in therapy.

A still further aspect is a pharmaceutical composition comprising 3-(3,4-dimethoxyphenyl)-2,6-dimethyl-N-(pyridin-4-ylmethyl)imidazo[1,2-b]pyridazin-8-amine or a pharmaceutically acceptable salt thereof and optionally a pharmaceutically acceptable excipient.

A further aspect is the use of a compound of formula (I) or a pharmaceutically acceptable salt thereof in the manufacture of a medicament for the treatment of a *Pneumoviridae* viral infection.

A further aspect is a pharmaceutical composition comprising a compound of formula (I) or a pharmaceutically acceptable salt thereof, and optionally a pharmaceutically acceptable excipient, for the treatment of a *Pneumoviridae* viral infection.

### BRIEF DESCRIPTION OF THE DRAWINGS

**Figure 1** is a bar chart showing the extracellular (EX) and cell-associated (CA) RSV yield (in Log₁₀ PFU/ml) obtained in HEp-2 cells by addition of 0.2 µM of the compound of either Example 25 or Example 61. Also shown is the corresponding RSV yield in the presence of vehicle (DMSO) only.
**Figure 2** is a bar chart showing the extracellular RSV yield (in Log₁₀ PFU/ml) obtained in HEp-2 cells by addition of 0.2 µM of the compound of either Example 25 or Example 61 at different time points (from -1h through 0h (beginning of infection), 2h (end of infection), 3h (1h after infection), 4h, 5h, and 6h relative to RSV infection of HEp-2 cells. ("Ctr" - No addition of compound)
**Figure 3** is a graph representing the plasma concentration (in ng/ml) and lung tissue concentration (in ng/g) of the compound of Example 68 after administration of a dose of 50 mg/kg per orally in Sprague Dawley rats.

### DETAILED DESCRIPTION OF THE INVENTION

"Pharmaceutically acceptable" means being useful in preparing a pharmaceutical composition that is generally safe, non-toxic and neither biologically nor otherwise undesirable and includes being useful for veterinary use as well as human pharmaceutical use.

The term "treating" (or "treatment") of a disease or disorder may refer to ameliorating the disease or disorder (i.e., arresting or reducing the development of the disease or at least one of the clinical symptoms thereof), and/or ameliorating at least one physical parameter, which may not be discernible by the patient. The term also may refer to inhibiting the disease or disorder, either physically, (e.g., stabilization of a discernible symptom), physiologically, (e.g., stabilization of a physical parameter), or both.

An "effective amount" refers to an amount of a compound that confers a therapeutic effect on the treated subject. The therapeutic effect may be objective (i.e., measurable by some test or marker, e.g. no measurable virus titer in a biological sample from the treated subject) or subjective (i.e., subject gives an indication of or feels an effect).

The term "excipient" refers to a pharmaceutically acceptable chemical, such as known to those of ordinary skill in the art of pharmacy to aid the administration of a medicinal agent. It is a compound that is useful in preparing a pharmaceutical composition, generally safe, non-toxic and neither biologically nor otherwise undesirable, and includes excipients that are acceptable for veterinary use as well as human pharmaceutical use. Exemplary excipients include binders, surfactants, diluents, disintegrants, anti adherents, and lubricants.

A "viral infection" refers to an infection by a virus, in a mammal.

A *"Pneumoviridae* viral infection" refers to an infection by a virus belonging to the taxonomic family *Pneumoviridae,* in a mammal. The viral family *Pneumoviridae* contains the two genuses *Metapneumovirus* and *Orthopneumovirus.*

An "orthopneumoviral infection" refers to an infection by a virus belonging to the *Orthopneumovirus* genus.

A "metapneumoviral infection" refers to an infection by a virus belonging to the *Metapneumovirus* genus.

An "RNA viral infection" refers to a viral infection wherein the virus has RNA (ribonucleic acid) as its genetic material.

An "enveloped single-stranded (-) RNA viral infection" refers to an infection by an enveloped single-stranded (-) RNA virus.

An "enveloped virus" is a virus having a viral envelope.

A "single-stranded (-) RNA virus" is a virus having genetic material which is single-stranded, negative-sense RNA, which RNA must first be converted to positive-stranded RNA before being translated to viral protein by the cell infected by the virus.

The term "respiratory tract" as used herein refers to the respiratory organs of a patient (bird or mammal), and includes the upper respiratory tract and lower respiratory tract.

The "upper respiratory tract" includes the nose and nasal passages, paranasal sinuses, the pharynx, and the portion of the larynx above the vocal folds.

The "lower respiratory tract" includes the trachea, bronchi, the bronchioles and lungs.

A "patient" as referred to herein may be an animal, e.g. a non-human mammal or a bird, or a human.

The term "mammal" refers to a human or any mammalian animal, e.g. a primate, a farm animal, a pet animal, or a laboratory animal. Examples of such animals are monkeys, cows, sheep, goats, horses, pigs, dogs, cats, rabbits, mice, rats etc. Preferably, the mammal is a human. In some embodiments, however, the mammal is an animal, e.g. a farm animal, such as a cow, sheep, goat, horse, or pig. In some other embodiments, the animal is a pet, e.g. a dog, a cat, or a rabbit.

A "bird" as referred to herein e.g. may be poultry, a cage bird or a pet bird.

Unless otherwise stated or indicated, the term "C1-C6 alkyl" denotes a straight or branched alkyl group having from 1 to 6 carbon atoms, e.g. methyl, ethyl, n-propyl, iso-propyl, n-butyl, iso-butyl, sec-butyl, t-butyl and straight- and branched-chain pentyl and hexyl.

Unless otherwise stated or indicated, the term "halogen" (or "halo") refers to fluorine (F), chlorine (Cl), or bromine (Br).
A moiety of the type RO is a moiety of formula 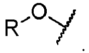
A moiety of the type RR'NC(O) is a moiety of formula 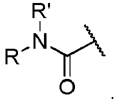
A moiety of the type RC(O)N(R') is a moiety of formula 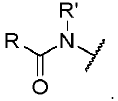
A moiety of the type RS(O)₂ is a moiety of formula 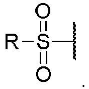
A moiety of the type RS(O)₂N(R') is a moiety of formula 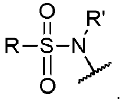
A moiety of the type RR'NS(O)₂ is a moiety of formula 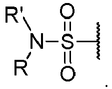
A moiety of the type RR'N is a moiety of formula 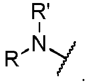
A moiety of the type RC(O) is a moiety of formula 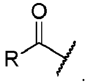
A moiety of the type ROC(O) is a moiety of formula 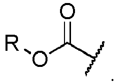
A moiety of the type RC(O)O is a moiety of formula 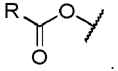
A moiety of the type RC(O)N(R')S(O)₂, is a moiety of formula 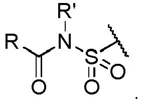
A moiety of the type and ROC(O)N(R')S(O)₂ is a moiety of formula 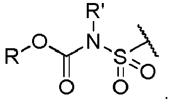
A moiety of the type -O⁻ is a covalently bound oxygen anion, i.e. a moiety of formula 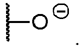
A moiety CN may also be referred to as "cyano" and is a moiety of formula

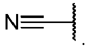

The term "heterocyclic ring" refers to a saturated or unsaturated, aromatic or non-aromatic cyclic moiety containing not only carbon atoms, but also at least one other atom in the ring, e.g. selected from nitrogen (N), sulphur (S) and oxygen (O). When aromatic, the heterocyclyl also may be referred to as "heteroaryl", which refers to an aromatic ring containing at least one ring heteroatom, such as furyl, isoxazolyl, isothiazolyl, imidazolyl, pyridyl, pyrrolyl, pyrazolyl, pyrimidinyl, pyridazinyl, pyrazinyl, oxadiazolyl, oxazolyl, thienyl, thiadiazolyl, thiazolyl, triazolyl, and tetrazolyl.

The term "aromatic", as used herein, refers to an unsaturated cyclic moiety that has an aromatic character, while the term "non-aromatic", as used herein, refers to a cyclic moiety, that may be saturated or unsaturated, e.g. polyunsaturated, but that does not have an aromatic character.
The term "benzyl" refers to a moiety of formula C₆H₅CH₂-, i.e.; 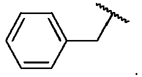
The term "pyridyl" (or pyridinyl) refers to a moiety of formula 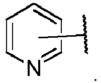
The term "3-pyridyl" (or "pyridin-3-yl") refers to a moiety of formula 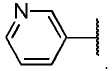
A 3-pyridyl substituted with a moiety R₂ in 6-position (e.g. 6-methyl-3-pyridyl) is a compound of formula 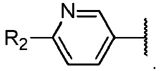
The term "4-pyridyl" (or pyridin-4-yl) refers to a moiety of formula 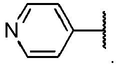
The term "thienyl" refers to a moiety of formula 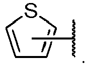
The term "2-thienyl" refers to a moiety of formula 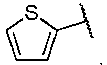
The term "furyl" refers to a moiety of formula 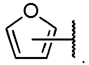
The term "2-furyl" refers to a moiety of formula

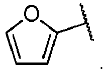

In a compound of formula (I), X is CH, Y is C, and Z is N; or X is CH, Y is N, and Z is C; or X is N, Y is N, and Z is C.

In some embodiments, X is CH, Y is C, and Z is N; or X is CH, Y is N, and Z is C; i.e. the compound may be represented by formula (Ia) 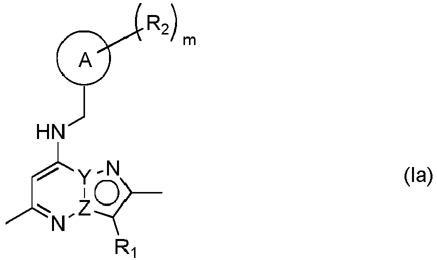 wherein Y, Z, R₁, R₂, ring A and m are as defined herein.

In some embodiments, X is CH, Y is N, and Z is C; or X is N, Y is N, and Z is C. In some embodiments, X is CH, Y is C, and Z is N; or X is N, Y is N, and Z is C.

In some embodiments, X is CH, Y is N, and Z is C. In these embodiments, the compound may be represented by formula (Ib) 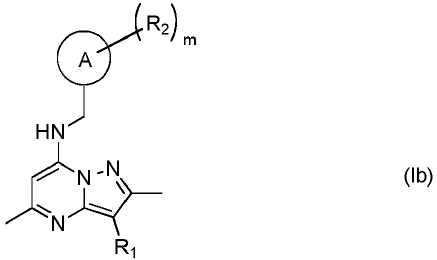 wherein R₁, R₂, ring A and m are as defined herein.

In some other embodiments, X is CH, Y is C, and Z is N. In these embodiments, the compound may be represented by formula (Ic) 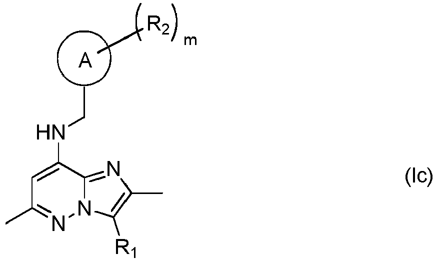 wherein R₁, R₂, ring A and m are as defined herein.

In some embodiments, X is N, Y is N, and Z is C. In these embodiments, the compound may be represented by formula (Id) 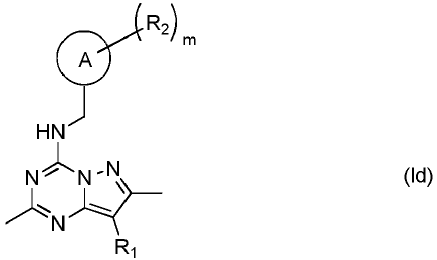 wherein R₁, R₂, ring A and m are as defined herein.

The moiety R₁ is 3,4-dimethoxyphenyl or 1,3-dimethyl-1H-indazol-5-yl. In some embodiments, R₁ is 3,4-dimethoxyphenyl, i.e. the compound may be represented by formula (Ie) 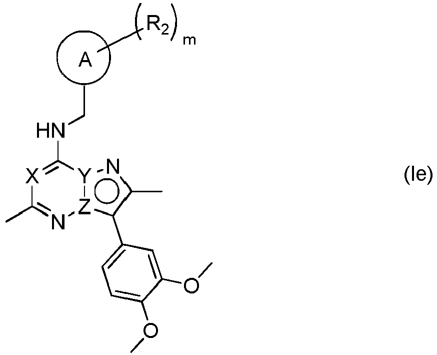 wherein X, Y, Z, R₂, ring A and m are as defined herein.

In some embodiments, R₁ is 1,3-dimethyl-1H-indazol-5-yl, i.e. the compound may be represented by formula (If) 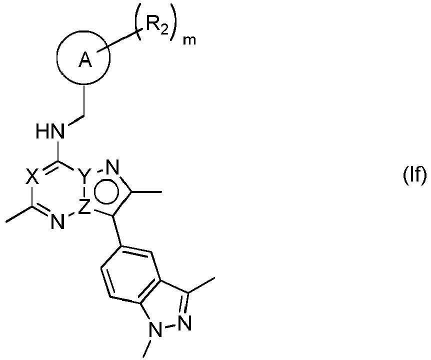 wherein X, Y, Z, R₂, ring A and m are as defined herein.

The compound of formula (I) comprises a moiety of formula (II) 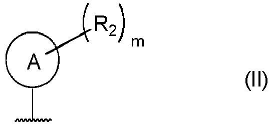 wherein R₂, ring A and m are as defined herein.

In the moiety of formula (II), ring A is phenyl or 5- or 6-membered heteroaryl. The heteroaryl preferably has one or more heteroatoms selected from O, S, and N. In some embodiments, ring A is 5- or 6-membered heteroaryl. In some other embodiments, ring A is phenyl or 6-membered heteroaryl. In still other embodiments, ring A is phenyl. In still other embodiments, ring A is phenyl or 5-membered heteroaryl. In some embodiments, ring A is 5-membered heteroaryl. In some other embodiments, ring A is 6-membered heteroaryl.

When ring A is 5- or 6-membered heteroaryl, said heteroaryl contains one or more heteroatoms. For example, said heteroaryl may contain 1, 2, 3, or 4 heteroatoms. In some embodiments, said heteroaryl contains 1, 2 or 3 heteroatoms. In some other embodiments, said heteroaryl contains or 1 or 2 heteroatoms. In still other embodiments, said heteroaryl contains 1 heteroatom.

Each heteroatom is independently selected from N, O and S. In some embodiments, each heteroatom is independently selected from N and O. In still other embodiments each heteroatom is independently selected from N and S. In some particular embodiments, at least one heteroatom is N, e.g. each heteroatom is N. In some embodiments, the heteroaryl contains one heteroatom, which is N. In some other embodiments, the heteroraryl contains one heteroatom, which is O. In some other embodiments, the heteroaryl contains one heteroatom, which is S.

In some embodiments, when ring A is 5-membered heteroaryl, said heteroaryl contains one heteroatom selected from O and S, and optionally one or more nitrogen atoms, e.g. 1-3 N, or 1 or 2 N, e.g. 1 N. In some embodiments, said heteroaryl is thienyl or furyl, e.g. 2-thienyl or 2-furyl. In some embodiments, when ring A is 5-membered heteroaryl, said heteroaryl more particularly is thienyl, e.g. 2-thienyl.

In some embodiments, when ring A is 6-membered heteroaryl, each heteroatom of said heteroaryl is N. For example, in some embodiments, when ring A is 6-membered heteroaryl, said heteroaryl is pyridyl, e.g. 3-pyridyl or 4-pyridyl, in particular 4-pyridyl.

In some embodiments, when ring A is 5- or 6-membered heteroaryl, said heteroaryl is selected from thienyl, furyl, pyrrolyl, imidazolyl, pyrazolyl, thizaolyl, isothiazolyl, oxazolyl, isoxazolyl, triazolyl, tetrazolyl, oxadiazolyl, thiadiazolyl, pyridyl, pyrazinyl, pyrimidinyl, and pyridazinyl.

In some embodiments, when ring A is phenyl or 6-membered heteroaryl, the moiety of formula (II) more particularly is a moiety of formula (IIa) 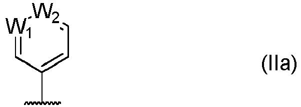 wherein W₁ and W₂ are independently selected from N, CH and CR₂. In such embodiments, the compound may be represented by formula (Ig) 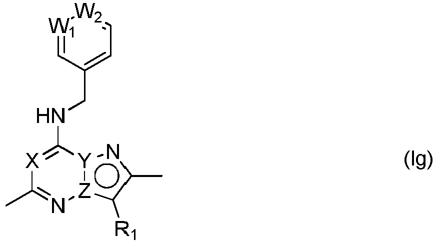 wherein X, Y, Z, R₁, W₁ and W₂ are as defined herein.

In some embodiments, one of W₁ and W₂ is different from N. In some embodiments, at least one of W₁ and W₂ is N. In some embodiments, at least one of W₁ and W₂ is different from CH. In some embodiments, at least one of W₁ and W₂ is different from CR₂. In some embodiments, W₁ and W₂ are different from each other, i.e. W₁ and W₂ are not both N, not both CH and not both CR₂. In some particular embodiments, W₁ is as defined herein and W₂ is N or CR₂, in particular N.

In some further embodiments, the moiety of formula (II) is selected from a moiety comprising 5-membered heteroaryl, e.g. unsubstituted (m is 0) 5-membered heteroaryl, and a ring of formula (IIa) as defined herein; e.g. from unsubstituted 2-thienyl and a ring of formula (IIa) wherein W₁ is CH and W₂ is CR₂, or W₁ is CH and W₂ is N, or W₁ is N and W₂ is CR₂. In some further embodiments, the moiety of formula (II) is selected from unsubstituted 2-thienyl and a ring of formula (IIa) wherein W₁ is CH and W₂ is N, or W₁ is N and W₂ is CR₂.

In some particular embodiments, the moiety of formula (IIa) is selected from 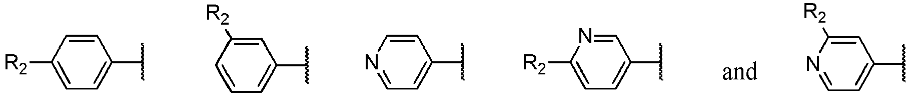 wherein R₂ is as defined herein.

In some embodiments, W₁ is CH or CR₂, and W₂ is N; or W₁ is N, and W₂ is CH₃; i.e. the moiety of formula (IIa) is selected from 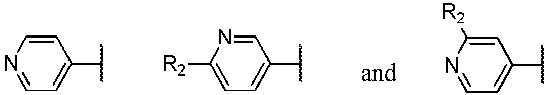 wherein R₂ is as defined herein.

In some further embodiments, W₁ is CH or CR₂, and W₂ is N, i.e. the moiety of formula (IIa) is selected from 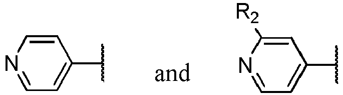 wherein R₂ is as defined herein, e.g. R₂ is selected from C1-C6 alkyl (including C1-C6 alkyl substituted by one or more fluoro), halogen, R₃O, and CN.

In some further embodiments, W₁ is CR₂ and W₂ is N, i.e. the moiety of formula (IIa) is 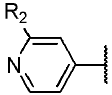 wherein R₂ is as defined herein, e.g. R₂ is selected from C1-C6 alkyl (including C1-C6 alkyl substituted by one or more fluoro), halogen, R₃O, and CN.

In still some further embodiments, W₁ is CH and W₂ is N.

In still some further embodiments, W₁ is CH or N, and W₂ is CR₂; or W₁ is CH and W₂ is N; i.e. the moiety of formula (IIa) is selected from 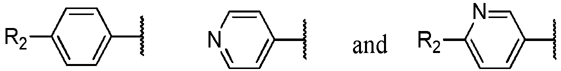 wherein R₂ is as defined herein.

In some further embodiments, W₁ is CH and W₂ is N; or W₁ is N and W₂ is CR₂, i.e. the moiety of formula (IIa) is selected from 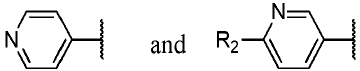 wherein R₂ is as defined herein.

In some further embodiments, W₁ is CH and W₂ is CR₂; or W₁ is N and W₂ is CR₂, i.e. the moiety of formula (IIa) is a moiety selected from 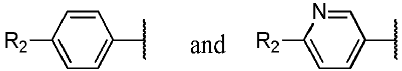 wherein R₂ is as defined herein.

In some further embodiments, W₁ is CH and W₂ is CR₂; or W₁ is CR₂ and W₂ is CH, i.e. the moiety of formula (IIa) is a moiety selected from 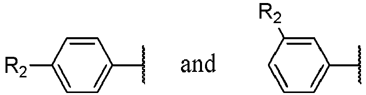 wherein R₂ is as defined herein.

In some embodiments, W₁ is CH and W₂ is CR₂. In some further embodiments, W₁ is CR₂ and W₂ is CH.

In a compound of formula (I), m is an integer 0, 1, 2 or 3 that denotes the number of substituents on ring A, i.e. ring A may be unsubstituted (m is 0) or substituted by 1, 2 or 3 moieties R₂ (m is 1, 2 or 3). In some embodiments, m is 0, 1 or 2. In some embodiments, m is 0 or 1. In some other embodiments, m is 0. In still other embodiments, m is 1, 2 or 3; or 1 or 2; in particular m is 1.

In some embodiments, when ring A is phenyl, m is different from 0. Thus, in some embodiments, when ring A is phenyl, m is as indicated herein above, but is not 0, e.g. m is 1 or 2, and when ring A is 5- or 6-membered heteroaryl, m is as indicated herein above, e.g. m is 0 or 1. In some other embodiments, when ring A is phenyl, m is 1, and when ring A is 5- or 6-membered heteroaryl, m is 0 or 1. In still further embodiments, when ring A is phenyl, m is 1, and when ring A is 5- or 6-membered heteroaryl, m is 0. In some further embodiments, when ring A is phenyl, m is 1, when ring A is 5-membered heteroaryl, m is 0, and when ring A is 6-membered heteroaryl, m is 0 or 1.

In some embodiments, ring A is phenyl and m is 1 or 2, or ring A is 5- or 6-membered heteroaryl and m is 0, 1 or 2. In some other embodiments, ring A is phenyl and m is 1, or ring A is 5- or 6-membered heteroaryl, and m is 0 or 1. In still further embodiments, ring A is phenyl, and m is 1, or ring A is 5- or 6-membered heteroaryl, and m is 0. In some further embodiments, ring A is phenyl, and m is 1, or ring A is 5-membered heteroaryl, and m is 0, or ring A is 6-membered heteroaryl, and m is 0 or 1.

In some embodiments, ring A is phenyl, and m is 0, 1 or 2, in particular 1 or 2. In some embodiments, ring A is phenyl, and m is 1.

In some embodiments, ring A is 5- or 6-membered heteroaryl, and m is 0 or 1. In some embodiments, ring A is 5- or 6-membered heteroaryl, and m is 0. In some embodiments, ring A is 5-membered heteroaryl, and m is 0. In some embodiments, ring A is 6-membered heteroaryl, and m is 0 or 1. In some embodiments, ring A is 6-membered heteroaryl, and m is 1. In some embodiments, ring A is 6-membered heteroaryl, and m is 0.

In some embodiments, when ring A is 3-pyridyl, m is 1 or 2, in particular 1, and one R₂ is attached in 6-position on the pyridyl ring.

It should be realized that, unless mutually exclusive, and whether illustrated herein or not, any combinations of the embodiments as illustrated by formulas (Ia) to (Ig) are contemplated within the scope of formula (I). For example, in some embodiments, a compound of formula (Ia) (e.g. a compound of formula (Ib), or a compound of formula (Ic)), also is a compound of formula (Ie). In some further embodiments, a compound of formula (Ia) (e.g. a compound of formula (Ib), or a compound of formula (Ic)), also is a compound of formula (If).

In some embodiments, a compound of formula (Ia) also is a compound of formula (Ig), i.e. a compound that may be represented by formula (Ih) 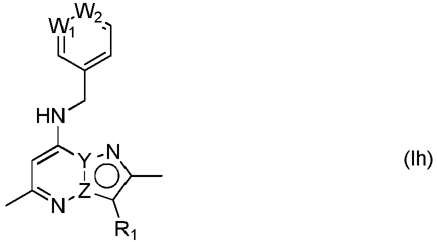 wherein Y, Z, R₁, W₁ and W₂ are as defined herein.

In some embodiments, a compound of formula (Ih) also is a compound of formula (Ie), i.e. the compound is one as represented by formula (Ij) 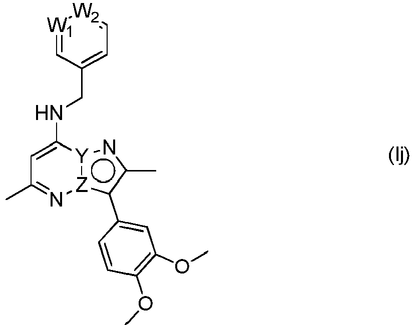 wherein Y, Z, W₁ and W₂ are as defined herein.

In still further embodiments, a compound of formula (Ij), also is a compound of formula (Ib), i.e. a compound as represented by formula (Ik) 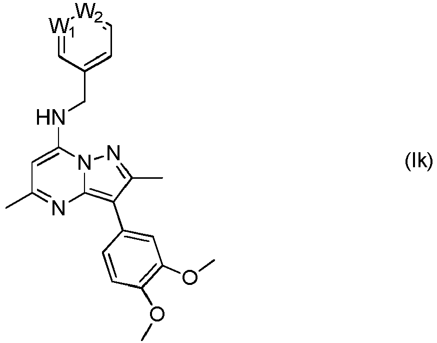 wherein W₁ and W₂ are as defined herein.

In some other embodiments, a compound of formula (Ih) also is a compound of formula (If), i.e. the compound is one as represented by formula (Im) 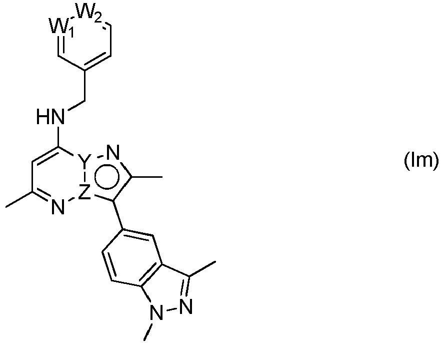 wherein Y, Z, W₁ and W₂ are as defined herein.

In still further embodiments, a compound of formula (Im), also is a compound of formula (Ib), i.e. a compound as represented by formula (In) 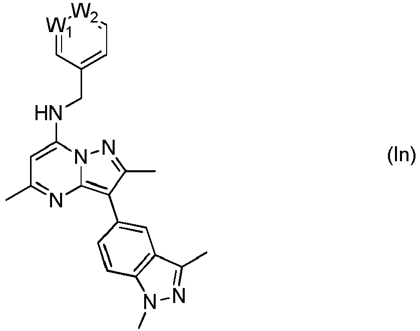 wherein W₁ and W₂ are as defined herein.

Disclosed herein is also the compound 3-(3,4-dimethoxyphenyl)-2,6-dimethyl-N-(pyridin-4-ylmethyl)imidazo[1,2-b]pyridazin-8-amine 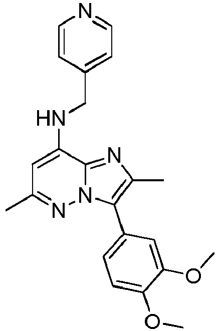 or a pharmaceutically acceptable salt thereof. This compound, which is a compound of formula (I), is novel and therefore claimed *per se,* as well as for use in therapy, in particular for the treatment of viral infections, such *Pneumoviridae* viral infections.

It should be realized that any reference made herein to a compound of formula (I) implicitly also is a reference to a compound of any of the embodiments of such a compound, e.g. as illustrated in any one of the formulas (Ia) to (In), unless otherwise indicated or apparent from the context.

In a compound of formula (I), each R₂ is independently selected from C1-C6 alkyl, halogen, R₃O, R₄R₅N, R₆R₇NS(O)₂, R₈R₉NC(O), R₁₀S(O)₂, R₁₁S(O)₂N(R₁₂), R₁₃C(O)O, R₁₄C(O)N(R₁₅), R₁₆OC(O), R₁₇C(O), R₁₈C(O)N(R₁₉)S(O)₂, R₂₀OC(O)N(R₂₁)S(O)₂, CN and -O⁻; and two R₂, attached to adjacent atoms of ring A, together with the atoms to which they are attached may form a 5- or 6-membered heterocyclic or carbocyclic ring.

In some embodiments, each R₂ is independently selected from C1-C6 alkyl, halogen, R₃O, R₄R₅N, R₆R₇NS(O)₂, R₁₀S(O)₂, R₁₁S(O)₂N(R₁₂), R₁₄C(O)N(R₁₅), R₁₇C(O), R₁₈C(O)N(R₁₉)S(O)₂, R₂₀OC(O)N(R₂₁)S(O)₂, CN and -O⁻; and two R₂, attached to adjacent atoms of ring A, together with the atoms to which they are attached may form a 5- or 6-membered heterocyclic or carbocyclic ring.

In some embodiments, each R₂ is independently selected from C1-C6 alkyl, halogen, R₃O, R₆R₇NS(O)₂, R₁₀S(O)₂, R₁₁S(O)₂N(R₁₂), and R₁₄C(O)N(R₁₅), and two R₂, attached to adjacent atoms of ring A, together with the atoms to which they are attached may form a 5- or 6-membered heterocyclic or carbocyclic ring.

In some embodiments, each R₂ is independently selected from C1-C6 alkyl, halogen, R₃O, R₆R₇NS(O)₂, R₁₀S(O)₂, and R₁₁S(O)₂N(R₁₂), and two R₂, attached to adjacent atoms of ring A, together which they are attached may form a 5- or 6-membered heterocyclic or carbocyclic ring.

In some embodiments, each R₂ is independently selected from C1-C6 alkyl, halogen, and R₃O, and two R₂, attached to adjacent atoms of ring A, together which they are attached may form a 5- or 6-membered heterocyclic or carbocyclic ring.

In some embodiments, each R₂ is independently selected from C1-C6 alkyl and R₃O, and two R₂, attached to adjacent atoms of ring A, together which they are attached may form a 5- or 6-membered heterocyclic or carbocyclic ring.

In some embodiments, each R₂ is independently selected from C1-C6 alkyl and halogen, and two R₂, attached to adjacent atoms of ring A, together which they are attached may form a 5- or 6-membered heterocyclic or carbocyclic ring.

In some further embodiments, each R₂ is independently selected from C1-C6 alkyl, halogen, R₃O, R₄R₅N, R₆R₇NS(O)₂, R₁₀S(O)₂, R₁₁S(O)₂N(R₁₂), R₁₄C(O)N(R₁₅), R₁₇C(O), R₁₈C(O)N(R₁₉)S(O)₂, and R₂₀OC(O)N(R₂₁)S(O)₂, and two R₂, attached to adjacent atoms of ring A, together with the atoms to which they are attached may form a 5- or 6-membered heterocyclic or carbocyclic ring.

In some further embodiments, each R₂ is independently selected from C1-C6 alkyl, halogen, R₃O, R₄R₅N, R₆R₇NS(O)₂, R₁₀S(O)₂, and R₁₁S(O)₂N(R₁₂), and two R₂, attached to adjacent atoms of ring A, together with the atoms to which they are attached may form a 5- or 6-membered heterocyclic or carbocyclic ring.

In some further embodiments, each R₂ is independently selected from C1-C6 alkyl, halogen, R₃O, R₆R₇NS(O)₂, R₁₀S(O)₂, and R₁₁S(O)₂N(R₁₂), and two R₂, attached to adjacent atoms of ring A, together with the atoms to which they are attached may form a 5- or 6-membered heterocyclic or carbocyclic ring.

In some embodiments, each R₂ is independently selected from C1-C6 alkyl, halogen, R₃O, R₄R₅N, R₆R₇NS(O)₂, R₈R₉NC(O), R₁₀S(O)₂, R₁₁S(O)₂N(R₁₂), R₁₃C(O)O, R₁₄C(O)N(R₁₅), R₁₆OC(O), R₁₇C(O), R₁₈C(O)N(R₁₉)S(O)₂, R₂₀OC(O)N(R₂₁)S(O)₂, CN and -O⁻.

In some embodiments, each R₂ is independently selected from C1-C6 alkyl, halogen, R₃O, R₄R₅N, R₆R₇NS(O)₂, R₁₀S(O)₂, R₁₁S(O)₂N(R₁₂), R₁₄C(O)N(R₁₅), R₁₇C(O), R₁₈C(O)N(R₁₉)S(O)₂, R₂₀OC(O)N(R₂₁)S(O)₂, CN and -O⁻.

In some embodiments, each R₂ is independently selected from C1-C6 alkyl, halogen, R₃O, R₆R₇NS(O)₂, R₁₀S(O)₂, R₁₁S(O)₂N(R₁₂), and R₁₄C(O)N(R₁₅).

In some embodiments, each R₂ is independently selected from C1-C6 alkyl, halogen, R₃O, R₆R₇NS(O)₂, R₁₀S(O)₂, and R₁₁S(O)₂N(R₁₂).

In some embodiments, each R₂ is independently selected from C1-C6 alkyl, R₆R₇NS(O)₂, R₁₀S(O)₂, and R₁₁S(O)₂N(R₁₂).

In some embodiments, each R₂ is independently selected from C1-C6 alkyl, R₆R₇NS(O)₂, and R₁₀S(O)₂.

In some embodiments, one R₂ is independently selected from R₆R₇NS(O)₂, and R₁₀S(O)₂.

In some embodiments, one R₂ is R₆R₇NS(O)₂.

In some embodiments, one R₂ is R₁₀S(O)₂.

In some embodiments, each R₂ is independently selected from C1-C6 alkyl, halogen, and R₃O.

In some embodiments, each R₂ is independently selected from C1-C6 alkyl and R₃O.

In some embodiments, each R₂ is independently selected from C1-C6 alkyl and halogen.

In some embodiments, each R₂ is independently selected from C1-C6 alkyl.

In some embodiments, each R₂ is independently selected from halogen.

In some further embodiments, each R₂ is independently selected from C1-C6 alkyl, halogen, R₃O, R₄R₅N, R₆R₇NS(O)₂, R₁₀S(O)₂, R₁₁S(O)₂N(R₁₂), R₁₄C(O)N(R₁₅), R₁₇C(O), R₁₈C(O)N(R₁₉)S(O)₂, and R₂₀OC(O)N(R₂₁)S(O)₂.

In some further embodiments, each R₂ is independently selected from C1-C6 alkyl, halogen, R₃O, R₄R₅N, R₆R₇NS(O)₂, R₁₀S(O)₂, and R₁₁S(O)₂N(R₁₂).

In some further embodiments, each R₂ is independently selected from C1-C6 alkyl, halogen, R₃O, R₆R₇NS(O)₂, R₁₀S(O)₂, and R₁₁S(O)₂N(R₁₂).

In some further embodiments, each R₂ is independently selected from C1-C6 alkyl, R₆R₇NS(O)₂, and R₁₀S(O)₂.

In some further embodiments, each R₂ is independently selected from C1-C6 alkyl, halogen, R₃O, and CN.

When R₂ is C1-C6 alkyl, it more particularly may be C1-C5 alkyl, or C1-C4 alkyl, or C1-C3 alkyl, or C1-C2 alkyl, e.g. methyl.

When R₂ is halogen, it e.g. may be selected from F, Cl, and Br, more particularly from F and Cl. In some embodiments, when R₂ is halogen, said halogen is F.

When R₂ comprises a moiety R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, and R₂₁, any such moiety is independently selected from H and C1-C6 alkyl. In some embodiments, any such moiety is independently selected from Hand C1-C5 alkyl, or from Hand C1-C4 alkyl, or from Hand C1-C3 alkyl, or from Hand C1-C2 alkyl, e.g. from H and methyl.

When any one of R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, and R₂₁, is a moiety selected from C1-C6 alkyl, such moiety more particularly may be selected from C1-C5 alkyl, or from C1-C4 alkyl, or from C1-C3 alkyl, or from C1-C2 alkyl, e.g. it may be methyl.

In some embodiments, each R₁₂, R₁₅, R₁₉, and R₂₁, when present, is H.

In some embodiments, each R₃, R₄, R₅, R₆, R₇, R₈, R₉, R₁₀, R₁₁, R₁₃, R₁₄, R₁₆, R₁₇, R₁₈, and R₂₀, when present, is selected from C1-C6 alkyl; and each R₁₂, R₁₅, R₁₉, and R₂₁, when present, is H.

In some embodiments, each R₃, R₄, R₅, R₆, R₇, R₈, R₉, R₁₃, and R₁₆, when present, is selected from H and C1-C6 alkyl; each R₁₀, R₁₁, R₁₄, R₁₇, R₁₈, and R₂₀, when present, is selected from C1-C6 alkyl; and each R₁₂, R₁₅, R₁₉, and R₂₁, when present, is H.

Any alkyl present in R₂, or as a moiety R₂, may optionally be substituted by one or more F, i.e. any alkyl may be or comprise a moiety selected from CF₃-, CF₂H-, CFH₂-, -CF₂-, -CFH-, and -CF<. In some embodiments, no such fluoro substitution is present in any alkyl group present in or as a moiety R₂.

In some embodiments, when m is at least 2, e.g. m is 2, two R₂ attached to adjacent atoms of the ring A, together with the atoms to which they are attached, form a 5- or 6-membered heterocyclic or carbocyclic ring. In some embodiments, the ring is carbocyclic. In some other embodiments, the ring is heterocyclic. In some embodiments, the ring is carbocyclic or 5-membered and heterocyclic.

In some embodiments, when two R₂ attached to adjacent atoms of the ring A form, together with the atoms to which they are attached, a 5- or 6-membered heterocyclic ring, said ring is a 5- membered heterocyclic ring, e.g. 1,3-dioxole or 1,3-dioxolane. In some embodiments, when two R₂ attached to adjacent atoms of the ring A, together with the atoms to which they are attached, form a carbocyclic ring, said ring is a benzene ring.

The compounds of formula (I) also may be transformed into suitable, pharmaceutically acceptable salts. The term pharmaceutically acceptable salt of a compound refers to a salt that is pharmaceutically acceptable, as defined herein, and that possesses the desired pharmacological activity of the parent compound. Pharmaceutically acceptable salts include acid addition salts formed with inorganic acids, e.g. hydrochloric acid, hydrobromic acid, sulphuric acid, nitric acid, phosphoric acid; or formed with organic acids, e.g. acetic acid, benzenesulfonic acid, benzoic acid, camphorsulfonic acid, citric acid, ethanesulfonic acid, fumaric acid, glucoheptonic acid, gluconic acid, glutamic acid, glycolic acid, hydroxynaphtoic acid, 2-hydroxyethanesulfonic acid, lactic acid, maleic acid, malic acid, malonic acid, mandelic acid, methanesulfonic acid, muconic acid, 2-naphthalenesulfonic acid, propionic acid, salicylic acid, succinic acid, tartaric acid, p-toluenesulfonic acid, trimethylacetic acid, etc.

In the preparation of acid addition salts, preferably such acids are used which form suitably therapeutically acceptable salts. Examples of such acids are hydrohalogen acids, sulfuric acid, phosphoric acid, nitric acid, aliphatic, alicyclic, aromatic or heterocyclic carboxylic or sulfonic acids, such as formic acid, acetic acid, propionic acid, succinic acid, glycolic acid, lactic acid, malic acid, tartaric acid, citric acid, ascorbic acid, maleic acid, hydroxymaleic acid, pyruvic acid, p-hydroxybenzoic acid, embonic acid, methanesulfonic acid, ethanesulfonic acid, hydroxyethanesulfonic acid, halogenbenzenesulfonic acid, toluenesulfonic acid or naphthalenesulfonic acid.

Whenever a chiral atom is present in a chemical structure, it is intended that all stereoisomers associated with that chiral atom are encompassed by the structure, unless otherwise specified. Using the Cahn-Ingold-Prelog RS notational system, any asymmetric atom may be present in the (R)- or (S)-configuration, and the compound may be present as a mixture of its stereoisomers, e.g. a racemic mixture, or one stereoisomer only, each being within the scope of the present invention.

The present invention includes pharmaceutical compositions for use in the treatment of a *Pneumoviridae* viral infection, comprising a compound of formula (I), or an individual isomer, racemic or non-racemic mixture of isomers or a pharmaceutically acceptable salt thereof, together with at least one pharmaceutically acceptable excipient, e.g. a carrier, and optionally other therapeutic and/or prophylactic ingredients.

A pharmaceutical composition according to the invention may be for topical (local) or systemic administration, e.g. for enteral administration, such as rectal or oral administration, or for parenteral administration to a patient (e.g. a human patient) and comprises a therapeutically effective amount of a compound according to the invention or a pharmaceutically acceptable salt thereof, as active ingredient, in association with a pharmaceutically acceptable excipient, e.g. a pharmaceutically acceptable carrier. The therapeutically effective amount of the active ingredient is as defined herein and depends e.g. on the patient, the body weight, the age, the individual condition, individual pharmacokinetic data, the disease to be treated and the mode of administration.

For enteral, e.g. oral, administration, the compounds of the invention may be formulated in a wide variety of dosage forms. The pharmaceutical compositions and dosage forms may comprise a compound or compounds of the present invention or pharmaceutically acceptable salt(s) thereof as the active component. The pharmaceutically acceptable carriers may be either solid or liquid. Solid form preparations include powders, tablets, pills, lozenges, capsules, cachets, suppositories, and dispersible granules. A solid carrier may be one or more substances which may also act as diluents, flavouring agents, solubilizers, lubricants, suspending agents, binders, preservatives, tablet disintegrating agents, or an encapsulating material. In powders, the carrier generally is a finely divided solid which is a mixture with the finely divided active component. In tablets, the active component generally is mixed with the carrier having the necessary binding capacity in suitable proportions and compacted in the shape and size desired. Suitable carriers include but are not limited to magnesium carbonate, magnesium stearate, talc, sugar, lactose, pectin, dextrin, starch, gelatine, tragacanth, methylcellulose, sodium carboxymethylcellulose, a low melting wax, cocoa butter, and the like. The formulation of the active compound may comprise an encapsulating material as carrier, providing a capsule in which the active component, with or without carriers, is surrounded by a carrier, which is in association with it.

Other forms suitable for oral administration include liquid form preparations including emulsions, syrups, elixirs, aqueous solutions, aqueous suspensions, or solid form preparations which are intended to be converted shortly before use to liquid form preparations. Emulsions may be prepared in solutions, for example, in aqueous propylene glycol solutions or may contain emulsifying agents, for example, such as lecithin, sorbitan monooleate, or acacia. Aqueous solutions can be prepared by dissolving the active component in water and adding suitable colorants, flavors, stabilizers, and thickening agents. Aqueous suspensions can be prepared by dispersing the finely divided active component in water with viscous material, such as natural or synthetic gums, resins, methylcellulose, sodium carboxymethylcellulose, and other well-known suspending agents. Solid form preparations include solutions, suspensions, and emulsions, and may contain, in addition to the active component, colorants, flavors, stabilizers, buffers, artificial and natural sweeteners, dispersants, thickeners, solubilising agents, and the like.

Exemplary compositions for rectal administration include suppositories which can contain, for example, a suitable non-irritating excipient, such as cocoa butter, synthetic glyceride esters or polyethylene glycols, which are solid at ordinary temperatures, but liquefy and/or dissolve in the rectal cavity to release the drug.

The compounds of the invention also may be administered parenterally, e.g. by inhalation, injection or infusion, e.g. by intravenous, intraarterial, intraosseous, intramuscular, intracerebral, intracerebroventricular, intrasynovial, intrasternal, intrathecal, intralesional, intracranial, intracutaneous and subcutaneous injection or infusion. Thus, for parenteral administration, the pharmaceutical compositions of the invention may be in the form of a sterile injectable or infusible preparation, for example, as a sterile aqueous or oleaginous suspension. This suspension may be formulated according to techniques known in the art using suitable dispersing or wetting agents (e.g., Tween 80), and suspending agents. The sterile injectable or infusible preparation may also be a sterile injectable or infusible solution or suspension in a non-toxic parenterally acceptable diluent or solvent. For example, the pharmaceutical composition may be a solution in 1,3-butanediol. Other examples of acceptable vehicles and solvents that may be employed in the compositions of the present invention include, but are not limited to, mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant.

Solutions for parenteral use also may contain suitable stabilizing agents, and if necessary, buffer substances. Suitable stabilizing agents include antioxidizing agents, such as sodium bisulfate, sodium sulfite or ascorbic acid, either alone or combined, citric acid and its salts and sodium EDTA. Parenteral solutions may also contain preservatives, such as benzalkonium chloride, methyl- or propyl-paraben, and chlorobutanol.

For inhalation or nasal administration, suitable pharmaceutical formulations are as particles, aerosols, powders, mists or droplets, e.g. with an average size of about 10 µm in diameter or less. For example, compositions for inhalation may be prepared as solutions in saline, employing benzyl alcohol or other suitable preservatives, absorption promoters to enhance bioavailability, fluorocarbons, and/or other solubilising or dispersing agents known in the art.

The pharmaceutical compositions of the invention also may be administered topically, to the skin or to a mucous membrane. For topical application, the pharmaceutical composition may be e.g. a lotion, a gel, a paste, a tincture, a transdermal patch, or a gel for transmucosal delivery.

The composition may be formulated as a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax and water.

Alternatively, the pharmaceutical composition may be formulated as a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and water.

The pharmaceutical compositions of this invention may also be topically applied to the lower intestinal tract by rectal suppository formulation or in a suitable enema formulation.

Suitable pharmaceutical excipients, e.g. carriers, and methods of preparing pharmaceutical dosage forms are described in Remington's Pharmaceutical Sciences, Mack Publishing Company, a standard reference text in art of drug formulation. The pharmaceutical compositions may comprise from approximately 1 % to approximately 95%, preferably from approximately 20% to approximately 90% of a compound of the invention, together with at least one pharmaceutically acceptable excipient.

In some embodiments, the formulation of the invention is a liposomal formulation. Liposomal formulations are well-known within the pharmaceutical fields, and are described e.g. in Remington, Essentials of Pharmaceutics, Ed. Linda Felton (Pharmaceutical Press 2012), pages 456-7 and in numerous other publications. Information on e.g. choice of suitable liposome formulations, suitable lipids, preparation methods, etc. is easily available to the person of ordinary skill in the art. Examples of lipids for liposome formation are phospholipids, sphingolipids, sterol lipids, and fatty acids. Lipids suitable for liposome formation may be purchased e.g. from Avanti^{®} Polar Lipids, Inc.

In general, the compounds of the invention will be administered in a therapeutically effective amount by any of the accepted modes of administration for agents that serve similar utilities. Suitable daily dosages typically ranges from 1 to 1000 mg, e.g. 1-500 mg, or 1-50 mg of the compound of formula (I), or an equivalent amount of a pharmaceutically acceptable salt thereof, depending upon numerous factors such as the severity of the disease to be treated, the age and relative health of the patient, the potency of the compound used, the route and form of administration, and the indication towards which the administration is directed, etc. One of ordinary skill in the art of treating such diseases will be able, without undue experimentation and in reliance upon personal knowledge and the disclosure of this application, to ascertain a therapeutically effective amount of the compounds of the present invention for a given disease. Compounds of the invention may be administered as pharmaceutical formulations including those suitable for enteral or parenteral administration. The preferred manner of administration is generally oral using a convenient daily dosage regimen which can be adjusted according to the degree of affliction.

The compounds of the present invention are contemplated as useful for the treatment of diseases caused by infection by a virus belonging to the *Pneumoviridae* family.

*Pneumoviridae* is a new virus family in the order Mononegavirales, derived from the paramyxoviral subfamily *Pneumovirinae.* Natural hosts include humans, cattle, and rodents. There are currently 5 species in the *Pneumoviridae* family, divided between 2 genera (Metapneumovirus and Orthopneumovirus). Pneumoviruses are pleomorphic, capable of producing spherical and filamentous enveloped virions (virus particles) that vary in size from 150 to 200 nm in diameter. The nucleocapsid consisting of a protein shell and viral nucleic acids has a helical symmetry. The genome is composed of negative-sense single-stranded RNA that is non-segmented. Viruses in this family are often associated with respiratory infections, and are transmitted through respiratory secretions.

In some embodiments, the infection is an orthopneumoviral infection. In some of these embodiments, the virus is human orthopneumovirus (sometimes also referred to herein as human respiratory syncytial virus, or HSRV).

In some other embodiments, the infection is a metapneumoviral infection. In some embodiments, the virus is human metapneumovirus (HMPV), i.e. the infection is human metapneumoviral infection.

In some of the above embodiments, the infection is a respiratory tract infection, e.g. a lower tract respiratory infection, which may be a serious disease in particular in infants and elderly people, as well as in patients suffering from reduced respiratory function, such as patent suffering from asthma or chronic obstructive pulmonary disease. In some embodiments, therefore, a compound of the present invention is for use in the treatment of *Pneumoviridae* viral infection in a patient in need of such treatment. In some embodiments, the patient is an infant, an elderly, or a patient suffering from reduced respiratory function.

Therefore, provided herein is a compound for use in the treatment of a disease or disorder linked to (or caused by) a *Pneumoviridae* viral infection.

In some embodiments, the disease is an infection of the upper respiratory tract. In some embodiments the disease is an infection of the lower respiratory tract. In some embodiments, the disease is bronchiolitis. In some embodiments, the disease is pneumonia.

The present invention consequently includes a compound of formula (I) for use in the treatment of any of the above mentioned diseases; a pharmaceutical composition for use in the treatment of any of the above mentioned diseases; the use of a compound of formula (I) in the manufacturing of a medicament for the treatment of any of the above mentioned diseases.

Preferably, the compound of formula (I) is used for the treatment of a human patient. In some embodiments, however, the compound of formula (I) is used for the treatment of an animal (i.e. non-human patient), such as an animal as mentioned herein.

Compounds of the invention may be prepared by the person of ordinary skill in the art using his knowledge within the field of chemical synthesis, in light of the illustrating non-limiting examples that will follow herein below and by referring to the literature within the field, e.g. using methods as described in international application No. PCT/EP2015/051177 (WO 2015/110491); international application No. PCT/EP2016/063383 (WO 2016/206999), and international application No. PCT/EP2018/058522 (WO 2018/185120).

Some examples of compounds for use according to the invention are shown in Table 1.

**Table 1**

| Ex | Name | Formula |
|---|---|---|
| 1 | 3-(3,4-dimethoxyphenyl)-2,6-dimethyl-N-(pyridin-4-ylmethyl)imidazo[1,2-b]pyridazin-8-amine | 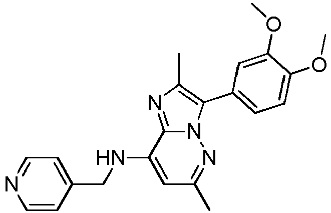 |
| 2 | 3-(3,4-dimethoxyphenyl)-2,6-dimethyl-N-((2-methylpyridin-4-yl)methyl)imidazo[1,2-b]pyridazin-8-amine | 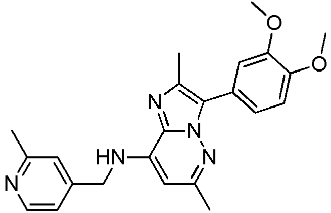 |
| 3 | 3-(3,4-dimethoxyphenyl)-N-((2-fluoropyridin-4-yl)methyl)-2,6-dimethylimidazo[1,2-b]pyridazin-8-amine | 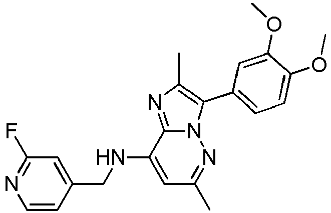 |
| 4 | 3-(1,3-dimethyl-1H-indazol-5-yl)-2,5-dimethyl-N-[(2-fluoropyridin-4-yl)methyl]pyrazolo[1,5-a]pyrimidin-7-amine | 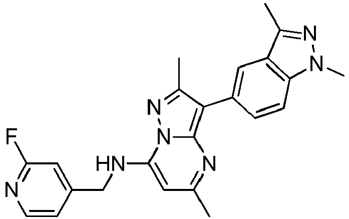 |
| 5 | 3-(1,3-dimethyl-1H-indazol-5-yl)-2,5-dimethyl-N-[(2-methylpyridin-4-yl)methyl]pyrazolo[1,5-a]pyrimidin-7-amine | 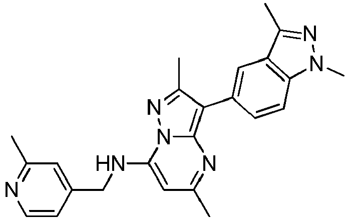 |
| 6 | 3-(1,3-dimethyl-1H-indazol-5-yl)-2,5-dimethyl-N-[(2-ethylpyridin-4-yl)methyl]pyrazolo[1,5-a]pyrimidin-7-amine | 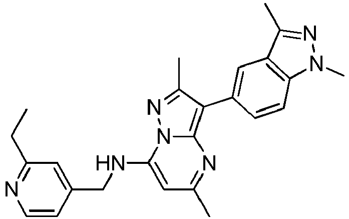 |
| 7 | 3-(1,3-dimethyl-1H-indazol-5-yl)-2,5-dimethyl-N-[(2-methoxypyridin-4-yl)methyl]pyrazolo[1,5-a]pyrimidin-7-amine | 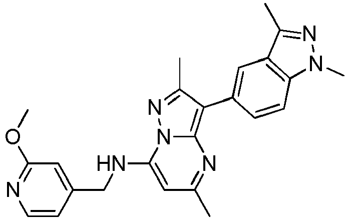 |
| 8 | 3-(1,3-dimethyl-1H-indazol-5-yl)-2,5-dimethyl-N-[(2-cyanopyridin-4-yl)methyl]pyrazolo[1,5-a]pyrimidin-7-amine | 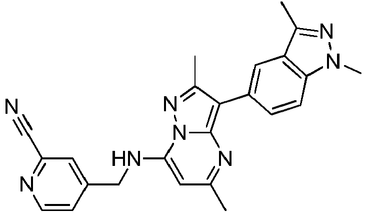 |
| 9 | 3-(1,3-dimethyl-1H-indazol-5-yl)-2,5-dimethyl-N-[(2-trifluoromethylpyridin-4-yl)methyl]pyrazolo[1,5-a]pyrimidin-7-amine | 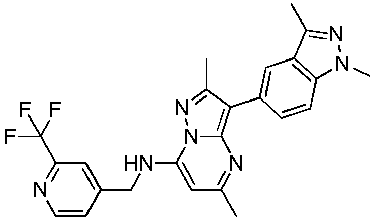 |
| 10 | N-[(4-chlorophenyl)methyl]-3-(3,4-dimethoxyphenyl)-2,5-dimethyl-pyrazolo[1,5-a]pyrimidin-7-amine | 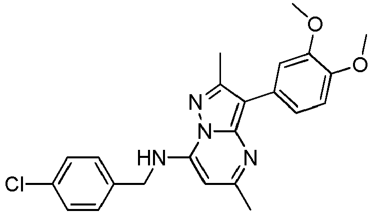 |
| 11 | 3-(3,4-dimethoxyphenyl)-N-[(4-methoxyphenyl)methyl]-2, 5-dimethylpyrazolo[1,5-a]pyrimidin-7-amine | 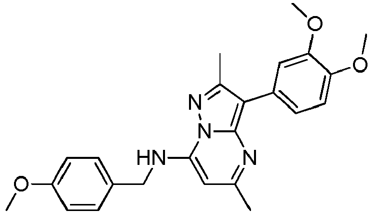 |
| 12 | 3-(3,4-dimethoxyphenyl)-2,5-dimethyl-N-(p-tolylmethyl)pyrazolo[1,5-a]pyrimidin-7-amine | 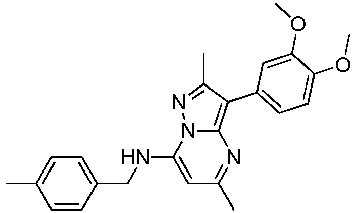 |
| 13 | N-(1,3-benzodioxol-5-ylmethyl)-3-(3,4-dimethoxyphenyl)-2,5-dimethyl-pyrazolo[1,5-a]pyrimidin-7-amine | 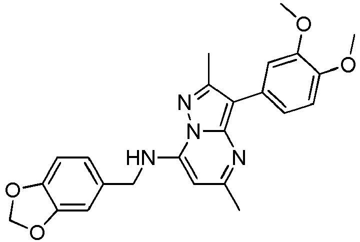 |
| 14 | 3-(3,4-dimethoxyphenyl)-N-[(4-fluorophenyl)methyl]-2, 5-dimethylpyrazolo[1,5-a]pyrimidin-7-amine | 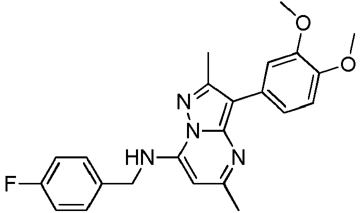 |
| 15 | 3-(3,4-dimethoxyphenyl)-2,5-dimethyl-N-(2-pyridylmethyl)pyrazolo[1,5-a]pyrimidin-7-amine | 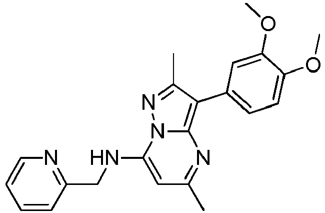 |
| 16 | N-benzyl-3-(3,4-dimethoxyphenyl)-2,5-dimethyl-pyrazolo[1,5-a]pyrimidin-7-amine | 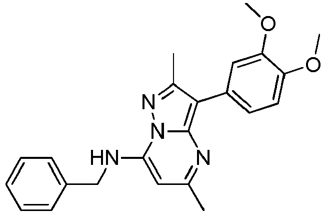 |
| 17 | 3-(3,4-dimethoxyphenyl)-2,5-dimethyl-N-[[4-(trifluoromethyl)phenyl]methyl]pyrazolo[1,5-a]pyrimidin-7-amine | 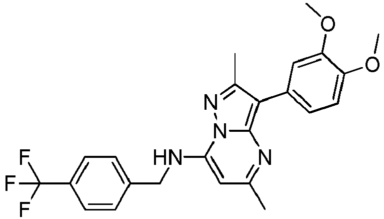 |
| 18 | 3-(3,4-dimethoxyphenyl)-2,5-dimethyl-N-(4-pyridylmethyl)pyrazolo[1,5-a]pyrimidin-7-amine | 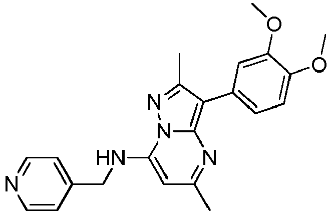 |
| 19 | 3-(3,4-dimethoxyphenyl)-2,5-dimethyl-N-[[4-(trifluoromethoxy)phenyl]methyl]pyrazolo[1, 5-a]pyrimidin-7-amine | 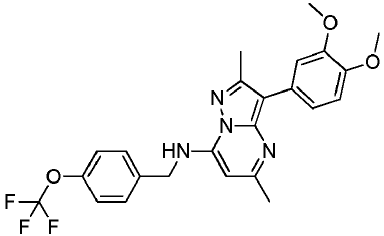 |
| 20 | N-[4-[[[3-(3,4-dimethoxyphenyl)-2,5-dimethyl-pyrazolo[1,5-a]pyrimidin-7-yl]amino]methyl]phenyl]acetamide | 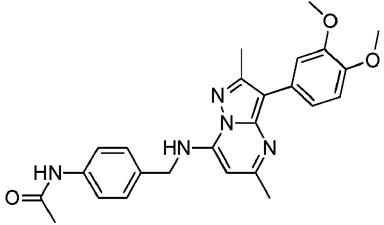 |
| 21 | 3-(3,4-dimethoxyphenyl)-N-[(4-dimethylaminophenyl)methyl]-2,5-dimethylpyrazolo[1,5-a]pyrimidin-7-amine | 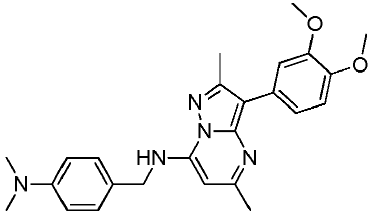 |
| 22 | 3-(3,4-dimethoxyphenyl)-N-[(6-methoxy-3-pyridyl)methyl]-2,5-dimethyl-pyrazolo[1,5-a]pyrimidin-7-amine | 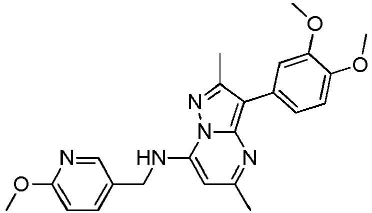 |
| 23 | N-[4-[[[3-(3,4-dimethoxyphenyl)-2,5-dimethyl-pyrazolo[1,5-a]pyrimidin-7-yl]amino]methyl]phenyl]methanesulfonamide | 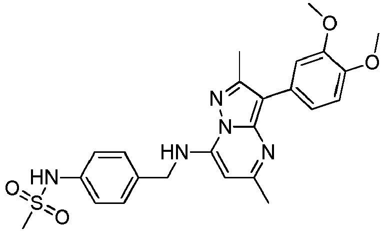 |
| 24 | 4-[[[3-(3,4-dimethoxyphenyl)-2,5-dimethylpyrazolo[1,5-a]pyrimidin-7-yl]amino]methyl]phenol | 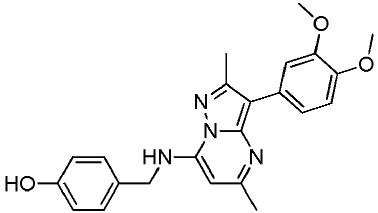 |
| 25 | 3-(3,4-dimethoxyphenyl)-2,5-dimethyl-N-[(3-methylsulfonylphenyl)methyl]pyrazolo[1,5-a]pyrimidin-7-amine | 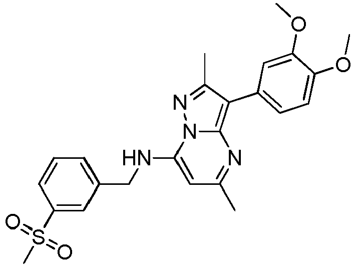 |
| 26 | 3-(3,4-dimethoxyphenyl)-2,5-dimethyl-N-[(4-methylsulfonylphenyl)methyl]pyrazolo[1,5-a]pyrimidin-7-amine | 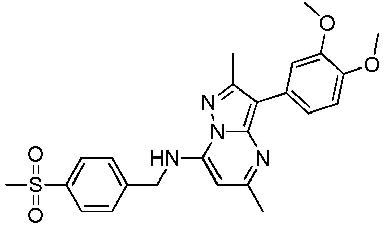 |
| 27 | N-[(4-tert-butylphenyl)methyl]-3-(3,4-dimethoxyphenyl)-2,5-dimethyl-pyrazolo[1,5-a]pyrimidin-7-amine | 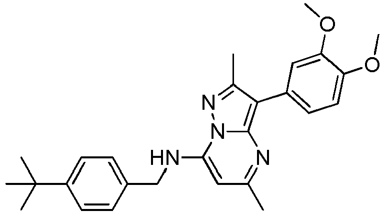 |
| 28 | 3-(3,4-dimethoxyphenyl)-2,5-dimethyl-N-[(2-methylpyrimidin-4-yl)methyl]pyrazolo[1,5-a]pyrimidin-7-amine | 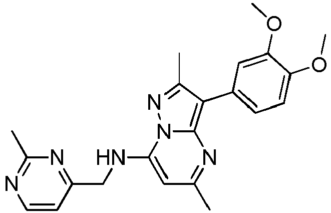 |
| 29 | 3-(3,4-dimethoxyphenyl)-2,5-dimethyl-N-[(6-methyl-3-pyridyl)methyl]pyrazolo[1,5-a]pyrimidin-7-amine | 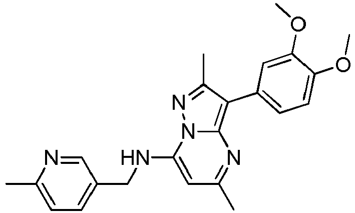 |
| 30 | 1-[4-[[[3-(3,4-dimethoxyphenyl)-2,5-dimethyl-pyrazolo[1,5-a]pyrimidin-7-yl]amino]methyl]phenyl]ethanone | 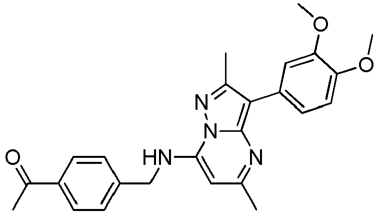 |
| 31 | 3-(3,4-dimethoxyphenyl)-2,5-dimethyl-N-(1-naphthylmethyl)pyrazolo[1,5-a]pyrimidin-7-amine | 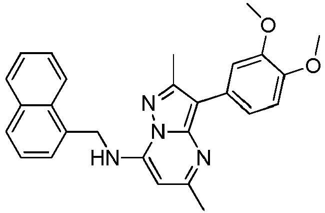 |
| 32 | 4-[[[3-(3,4-dimethoxyphenyl)-2,5-dimethylpyrazolo[1,5-a]pyrimidin-7-yl]amino]methyl]benzenesulfonamide | 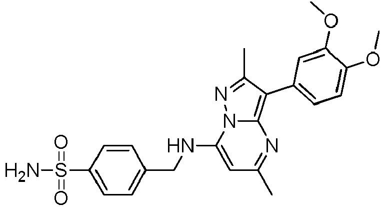 |
| 33 | 8-(3,4-dimethoxyphenyl)-N-[(4-fluorophenyl)methyl]-2,7-dimethylpyrazolo[1,5-a][1,3,5]triazin-4-amine | 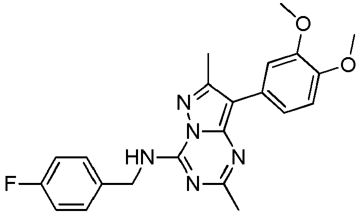 |
| 34 | 8-(3,4-dimethoxyphenyl)-2,7-dimethyl-N-[[4-(trifluoromethyl)phenyl]methyl]pyrazolo[1,5-a][1,3,5]triazin-4-amine | 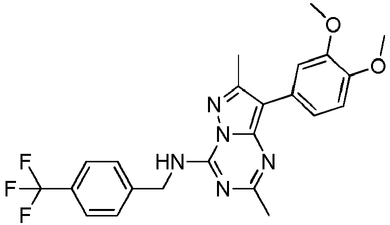 |
| 35 | 8-(3,4-dimethoxyphenyl)-2,7-dimethyl-N-(p-tolylmethyl)pyrazolo[1,5-a][1,3,5]triazin-4-amine | 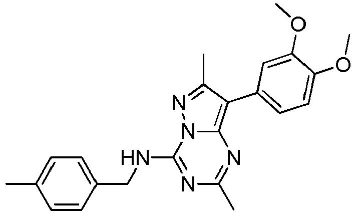 |
| 36 | 8-(3,4-dimethoxyphenyl)-N-[(4-isopropylphenyl)methyl]-2,7-dimethylpyrazolo[1,5-a][1,3,5]triazin-4-amine | 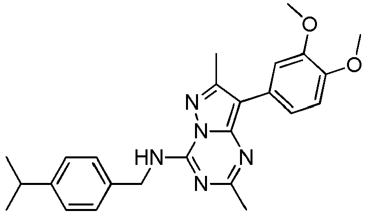 |
| 37 | 8-(3,4-dimethoxyphenyl)-2,7-dimethyl-N-(2-pyridylmethyl)pyrazolo[1,5-a][1,3,5]triazin-4-amine | 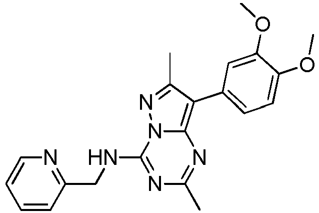 |
| 38 | 8-(3,4-dimethoxyphenyl)-2,7-dimethyl-N-(4-pyridylmethyl)pyrazolo[1,5-a][1,3,5]triazin-4-amine | 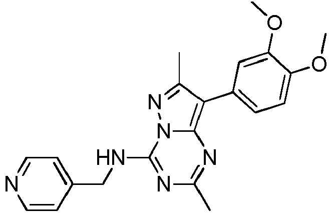 |
| 39 | N-[(4-chlorophenyl)methyl]-8-(3,4-dimethoxyphenyl)-2,7-dimethyl-pyrazolo[1,5-a][1,3,5]triazin-4-amine | 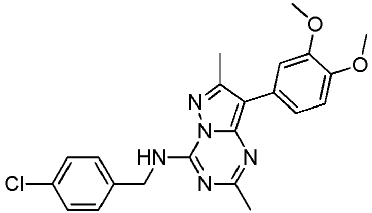 |
| 40 | N-[4-[[[8-(3,4-dimethoxyphenyl)-2,7-dimethyl-pyrazolo[1,5-a][1,3,5]triazin-4-yl]amino]methyl]phenyl]acetamide | 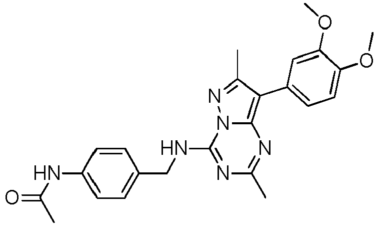 |
| 41 | 8-(3,4-dimethoxyphenyl)-N-[(4-methoxyphenyl)methyl]-2,7-dimethylpyrazolo[1,5-a][1,3,5]triazin-4-amine | 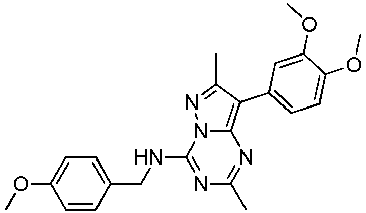 |
| 42 | 8-(3,4-dimethoxyphenyl)-2,7-dimethyl-N-[(3-methylsulfonylphenyl)methyl]pyrazolo[1,5-a][1,3,5]triazin-4-amine | 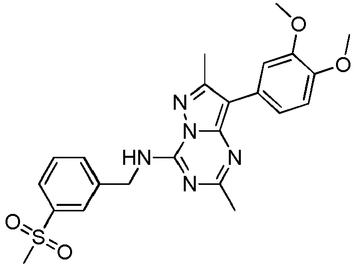 |
| 43 | 8-(3,4-dimethoxyphenyl)-2,7-dimethyl-N-[(1-oxidopyridin-1-ium-4-yl)methyl]pyrazolo[1,5-a][1,3,5]triazin-4-amine | 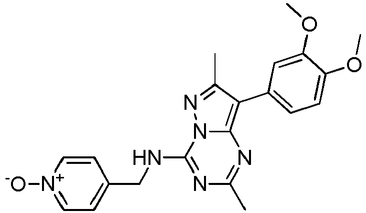 |
| 44 | 8-(3,4-dimethoxyphenyl)-N-[(6-methoxy-3-pyridyl)methyl]-2,7-dimethyl-pyrazolo[1,5-a][1,3,5]triazin-4-amine | 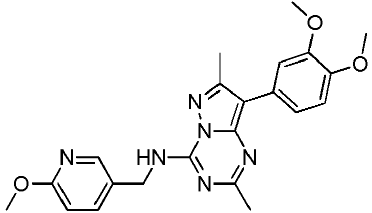 |
| 45 | 4-[[[8-(3,4-dimethoxyphenyl)-2,7-dimethylpyrazolo[1,5-a][1,3,5]triazin-4-yl]amino]methyl]benzenesulfonamide | 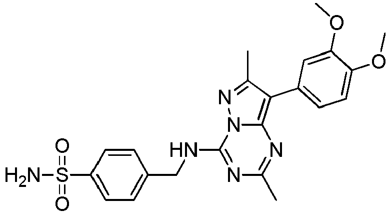 |
| 46 | N-(1,3-benzodioxol-5-ylmethyl)-8-(3,4-dimethoxyphenyl)-2,7-dimethyl-pyrazolo[1,5-a][1,3,5]triazin-4-amine | 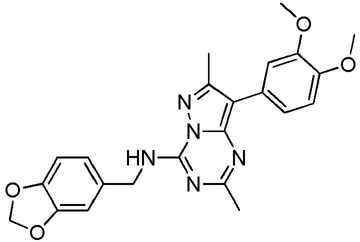 |
| 47 | 4-[[[8-(3,4-dimethoxyphenyl)-2,7-dimethylpyrazolo[1,5-a][1,3,5]triazin-4-yl]amino]methyl]phenol | 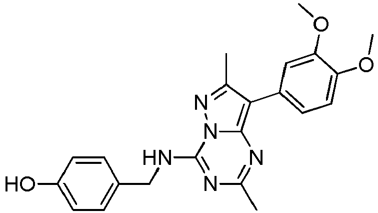 |
| 48 | N-[4-[[[8-(3,4-dimethoxyphenyl)-2,7-dimethyl-pyrazolo[1,5-a][1,3,5]triazin-4-yl]amino]methyl]phenyl]methanesulfonamide | 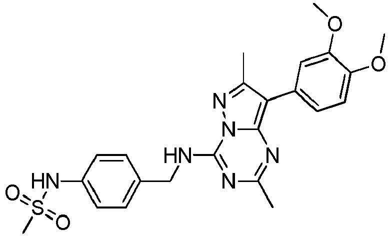 |
| 49 | N-benzyl-8-(3,4-dimethoxyphenyl)-2,7-dimethyl-pyrazolo[1,5-a][1,3,5]triazin-4-amine | 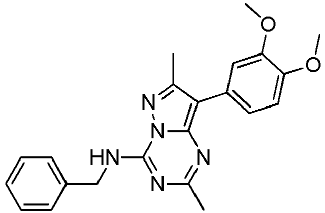 |
| 50 | 8-(3,4-dimethoxyphenyl)-2,7-dimethyl-N-(2-thienylmethyl)pyrazolo[1,5-a][1,3,5]triazin-4-amine | 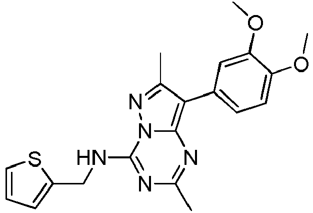 |
| 51 | 8-(3,4-dimethoxyphenyl)-2,7-dimethyl-N-(3-pyridylmethyl)pyrazolo[1,5-a][1,3,5]triazin-4-amine | 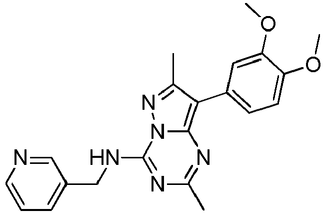 |
| 52 | 8-(3,4-dimethoxyphenyl)-2,7-dimethyl-N-[(5-methyl-2-furyl)methyl]pyrazolo[1,5-a][1,3,5]triazin-4-amine | 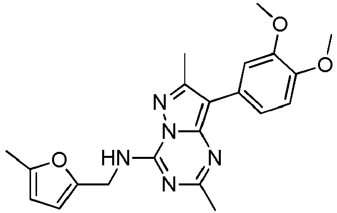 |
| 53 | methyl N-[4-[[[8-(3,4-dimethoxyphenyl)-2,7-dimethyl-pyrazolo[1,5-a][1,3,5]triazin-4-yl]amino]methyl]phenyl]sulfonylcarbamate | 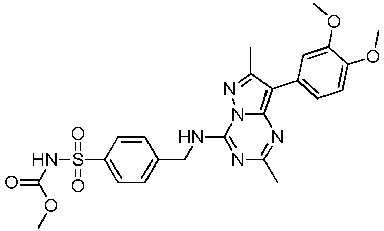 |
| 54 | N-[4-[[[8-(3,4-dimethoxyphenyl)-2,7-dimethyl-pyrazolo[1,5-a][1,3,5]triazin-4-yl]amino]methyl]phenyl]sulfonylpropanamide | 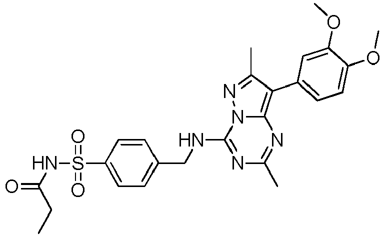 |
| 55 | N-methyl-4-[[[8-(3,4-dimethoxyphenyl)-2,7-dimethyl-pyrazolo[1,5-a][1,3,5]triazin-4-yl]amino]methyl]benzenesulfonamide | 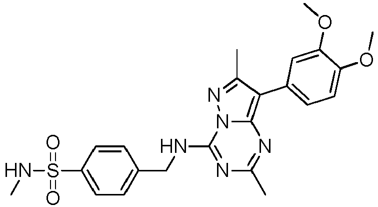 |
| 56 | 8-(3,4-dimethoxyphenyl)-2,7-dimethyl-*N*-[(1-methyl-1*H*-pyrazol-3-yl)methyl]pyrazolo[1,5-*a*][1,3,5]triazin-4-amine | 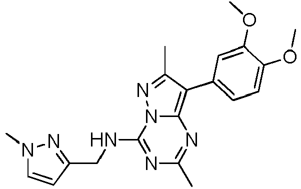 |
| 57 | 8-(3,4-dimethoxyphenyl)-2,7-dimethyl-*N-*[(1,3-oxazol-5-yl)methyl]pyrazolo[1,5-*a*][1,3,5]triazin-4-amine | 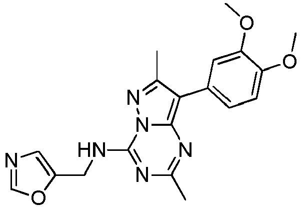 |
| 58 | 8-(3,4-dimethoxyphenyl)-2,7-dimethyl-*N*-[(2-methylpyrimidin-5-yl)methyl]pyrazolo[1,5-*a*][1,3,5]triazin-4-amine | 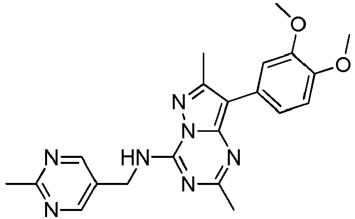 |
| 59 | 8-(3,4-dimethoxyphenyl)-2,7-dimethyl-*N-*[(pyridazin-4-yl)methyl]pyrazolo[1,5-*a*][1,3,5]triazin-4-amine | 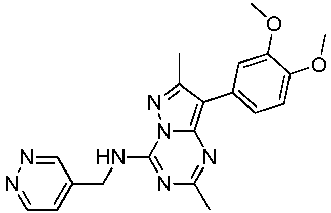 |
| 60 | 8-(3,4-dimethoxyphenyl)-2,7-dimethyl-*N*-[(6-methylpyridin-3-yl)methyl]pyrazolo[1,5-*a*][1,3,5]triazin-4-amine | 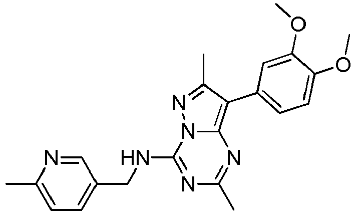 |
| 61 | 3-(1,3-dimethyl-1H-indazol-5-yl)-2,6-dimethyl-N-(pyridin-4-ylmethyl)imidazo[1,2-b]pyridazin-8-amine | 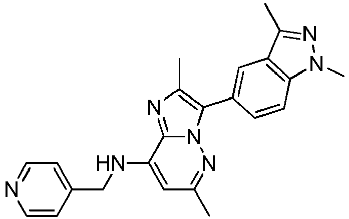 |
| 62 | 3-(1,3-dimethyl-1H-indazol-5-yl)-2,6-dimethyl-N-((6-methylpyridin-3 - yl)methyl)imidazo[1,2-b]pyridazin-8-amine | 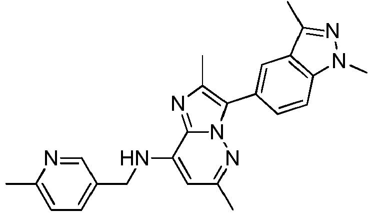 |
| 63 | N-(4-chlorobenzyl)-3 -(1,3 -dimethyl- 1H-indazol-5-yl)-2,6-dimethylimidazo[1,2-b]pyridazin-8-amine | 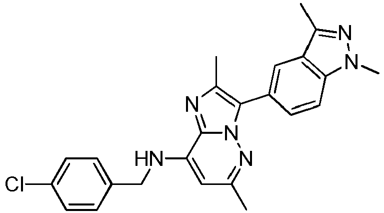 |
| 64 | 3-(1,3-dimethyl-1H-indazol-5-yl)-N-((6-methoxypyridin-3-yl)methyl)-2,6-dimethylimidazo[1,2-b]pyridazin-8-amine | 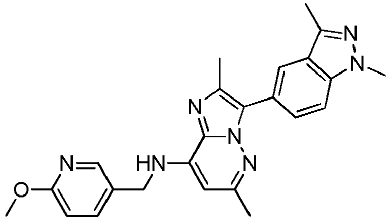 |
| 65 | 3-(1,3-dimethyl-1H-indazol-5-yl)-2,6-dimethyl-N-(thiophen-2-ylmethyl)imidazo[1,2-b]pyridazin-8-amine | 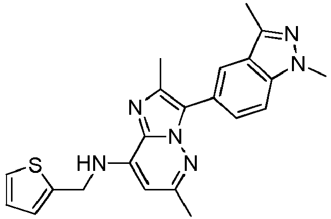 |
| 66 | 3-(1,3-dimethyl-1H-indazol-5-yl)-N-(4-methoxybenzyl)-2,6-dimethylimidazo[1,2-b]pyridazin-8-amine | 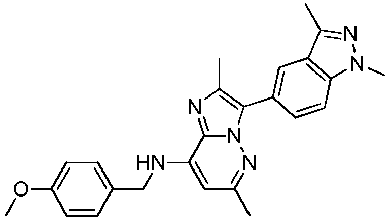 |
| 67 | 3-(1,3-dimethyl-1H-indazol-5-yl)-2,6-dimethyl-N-(4-(methylsulfonyl)benzyl)imidazo[1,2-b]pyridazin-8-amine | 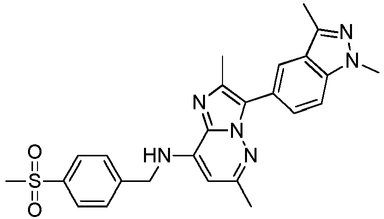 |
| 68 | 3-(1,3-dimethyl-1H-indazol-5-yl)-2,5-dimethyl-N-(pyridin-4-ylmethyl)pyrazolo[1,5-a]pyrimidin-7-amine | 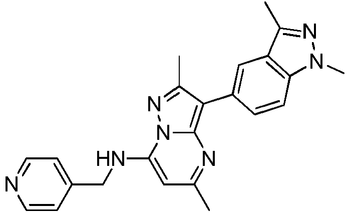 |
| 69 | 3-(1,3-dimethyl-1H-indazol-5-yl)-2,5-dimethyl-N-((6-methylpyridin-3 - yl)methyl)pyrazolo[1,5-a]pyrimidin-7-amine | 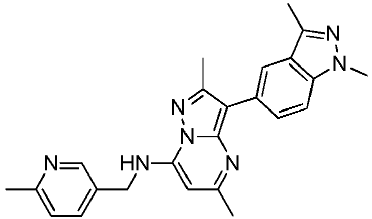 |
| 70 | 3-(1,3-dimethyl-1H-indazol-5-yl)-N-(4-methoxybenzyl)-2,5-dimethylpyrazolo[1,5-a]pyrimidin-7-amine | 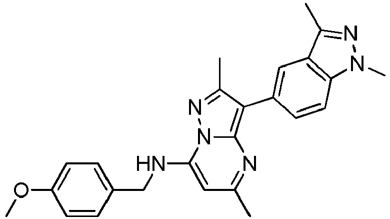 |
| 71 | N-(4-chlorobenzyl)-3 -(1,3 -dimethyl- 1H-indazol-5-yl)-2,5-dimethylpyrazolo[1,5-a]pyrimidin-7-amine | 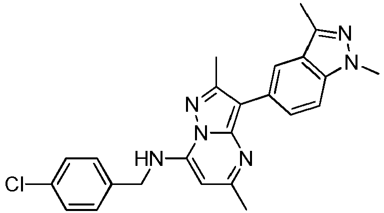 |
| 72 | 3-(1,3-dimethyl-1H-indazol-5-yl)-2,5-dimethyl-N-(4-(methylsulfonyl)benzyl)pyrazolo[1,5-a]pyrimidin-7-amine | 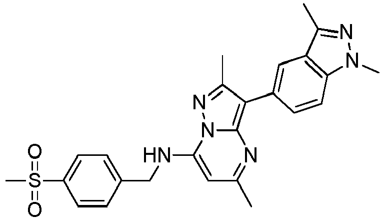 |
| 73 | 4-(((3-(1,3-dimethyl-1H-indazol-5-yl)-2,5-dimethylpyrazolo[1,5-a]pyrimidin-7-yl)amino)methyl)benzenesulfonamide | 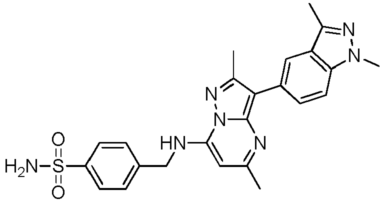 |
| 74 | 3-(1,3-dimethyl-1H-indazol-5-yl)-N-((6-methoxypyridin-3-yl)methyl)-2,5-dimethylpyrazolo[1,5-a]pyrimidin-7-amine | 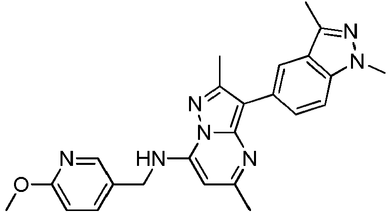 |
| 75 | 3-(1,3-dimethyl-1H-indazol-5-yl)-2,5-dimethyl-N-(thiophen-2-ylmethyl)pyrazolo[1,5-a]pyrimidin-7-amine | 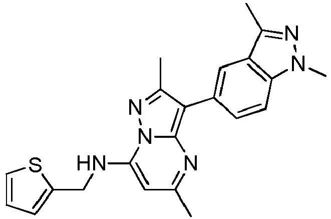 |
| 76 | N-(4-(((3-(1,3-dimethyl-1H-indazol-5-yl)-2,5-dimethylpyrazolo[1,5-a]pyrimidin-7-yl)amino)methyl)phenyl)methanesulfonamide | 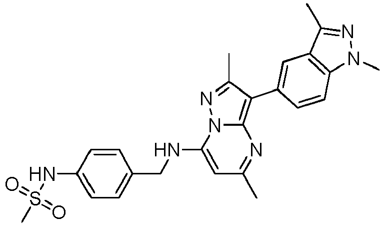 |
| 77 | 3-(1,3-dimethyl-1H-indazol-5-yl)-2,5-dimethyl-N-((6-trifluoromethylpyridin-3-yl)methyl)pyrazolo[1,5-a]pyrimidin-7-amine | 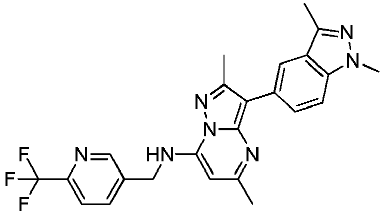 |
| 78 | N-(benzyl)-3-(1,3-dimethyl-1H-indazol-5-yl)-2,5-dimethylpyrazolo[1,5-a]pyrimidin-7-amine | 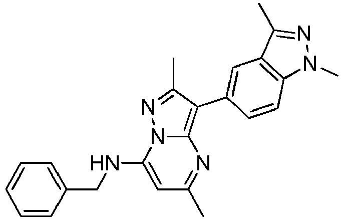 |
| 79 | N-(4-fluorobenzyl)-3-(1,3-dimethyl-1H-indazol-5-yl)-2,5-dimethylpyrazolo[1,5-a]pyrimidin-7-amine | 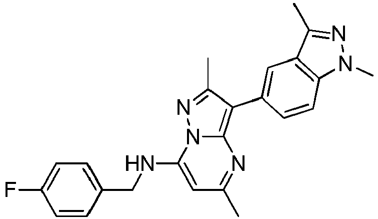 |
| 80 | 8-(1,3-dimethyl-1H-indazol-5-yl)-2,7-dimethyl-N-(pyridin-4-ylmethyl)pyrazolo[1,5-a][1,3,5]triazin-4-amine | 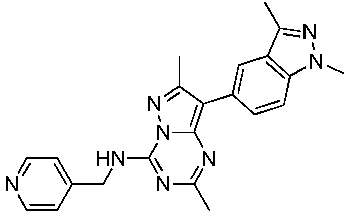 |
| 81 | 8-(1,3-dimethyl-1H-indazol-5-yl)-N-(4-methoxybenzyl)-2,7-dimethylpyrazolo[1,5-a][1,3,5]triazin-4-amine | 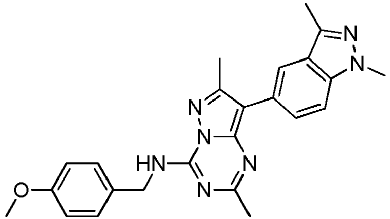 |
| 82 | 8-(1,3-dimethyl-1H-indazol-5-yl)-2,7-dimethyl-N-((6-methylpyridin-3 - yl)methyl)pyrazolo[1,5-a][1,3,5]triazin-4-amine | 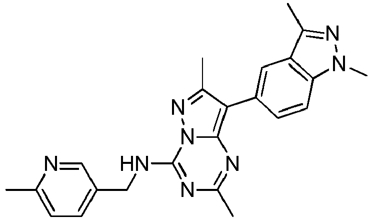 |
| 83 | 4-(((8-(1,3-dimethyl-1H-indazol-5-yl)-2,7-dimethylpyrazolo[1,5-a][1,3,5]triazin-4-yl)amino)methyl)benzenesulfonamide | 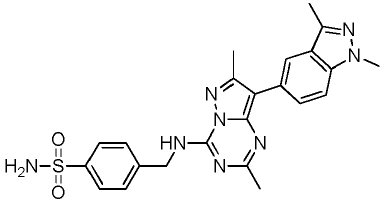 |
| 84 | N-(4-chlorobenzyl)-8-(1,3-dimethyl-1H-indazol-5-yl)-2,7-dimethylpyrazolo[1,5-a][1,3,5]triazin-4-amine | 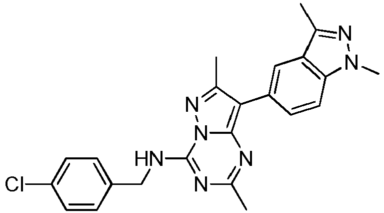 |
| 85 | 8-(1,3-dimethyl-1H-indazol-5-yl)-2,7-dimethyl-N-(4-(methylsulfonyl)benzyl)pyrazolo[1,5-a][1,3,5]triazin-4-amine | 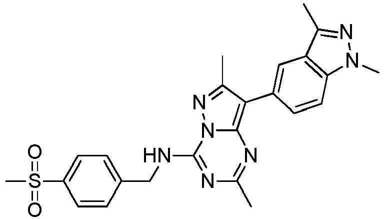 |
| 86 | N-(4-(((8-(1,3-dimethyl-1H-indazol-5-yl)-2,7-dimethylpyrazolo[1,5-a][1,3,5]triazin-4-yl)amino)methyl)phenyl)methanesulfonamide | 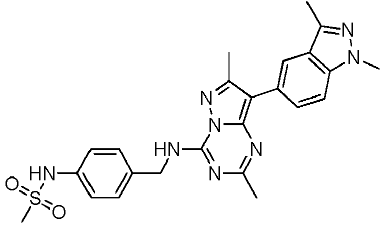 |
| 87 | 8-(1,3-dimethyl-1H-indazol-5-yl)-2,7-dimethyl-N-(thiophen-2-ylmethyl)pyrazolo[1,5-a][1,3,5]triazin-4-amine | 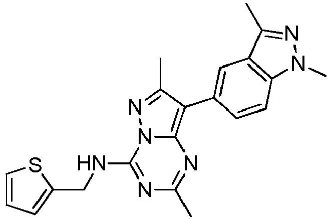 |
| 88 | 8-(1,3-dimethyl-1H-indazol-5-yl)-N-((6-methoxypyridin-3-yl)methyl)-2,7-dimethylpyrazolo[1,5-a][1,3,5]triazin-4-amine | 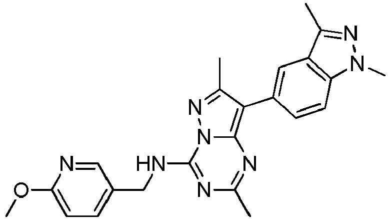 |

In the above Table 1, Example 1 is a novel compound, while compounds of Examples 2 to 88 are previously known. The synthesis and identification of the compound of Example 1 are described herein below. Additionally, the synthesis and identification of Examples 2-9 are described herein and are also described in international application No. PCT/EP2019/072220 (WO 2020/074159). The synthesis and identification of Examples 10-32 were described in international application No. PCT/EP2015/051177 (WO 2015/110491). The synthesis and identification of Examples 33-60 were described in international application No. PCT/EP2016/063383 (WO 2016/206999). The synthesis and identification of Examples 61-88 have been previously described in international application No. PCT/EP2018/058522 (WO 2018/185120).

### EXAMPLES

### General Procedures

Reactions were performed in flame-dried sealed-tubes or oven-dried glassware under a positive pressure of argon or nitrogen, unless otherwise noted. Air- and moisture-sensitive liquids and solutions were transferred via syringe. Tetrahydrofuran (THF) was distilled from sodium/benzophenone-ketyl. Dichloromethane (CH₂Cl₂) was distilled from calcium hydride. All other chemicals were obtained from commercial vendors and were used without further purification unless noted otherwise. Molecular sieves were activated at 350°C and were crushed immediately prior to use, then flame-dried under vacuum. Reactions were monitored by thin layer chromatography (TLC) with 0.25-mm E. Merck pre-coated silica gel plates. Organic solutions were concentrated by rotary evaporation below 50 °C. Flash column chromatography was performed employing 60-120, 230-400 mesh silica gel and neutral alumina. Yields refer to chromatographically and spectroscopically pure compounds unless otherwise noted.

### Instrumentation

¹H and ¹³C spectra were recorded either on a Bruker AVANCE III HD 400 MHz spectrometer or Bruker AVANCE II 300 MHz spectrometer. Chemical shifts are expressed in parts per million (δ scale) downfield from tetramethylsilane and are referenced to the residual resonance in the NMR solvent (CHCl₃: δ 7.26 for 1H NMR, δ 77.16 for 13C NMR). LC-MS was performed on an Agilent XCT Ion Trap equipped with chemstation and Bruker daltonics software.

### Synthesis of 3-(3,4-dimethoxyphenyl)-8-iodo-2,6-dimethylimidazo[1,2-b]pyridazine

The synthetic intermediary 3-(3,4-dimethoxyphenyl)-8-iodo-2,6-dimethylimidazo[1,2-b]pyridazine was synthesized in a multistep process as follows:

### Step 1: 2,6-dimethylimidazo[1,2-b]pyridazine

To a stirred solution of 6-methylpyridazine-3-ylamine (50 g, 455.3 mmol) in ethanol (500 mL) was added chloroacetone (58 ml, 683 mmol) and the solution was heated at 85°C for 10h. Upon completion, the ethanol in the reaction was distilled out. The obtained crude product was purified by flash column chromatography (neutral alumina) eluting the required compound, *2,6-dimethylimidazo[1,2-b]pyridazine* (36 g, 53.4%) with 10% ethyl acetate-hexanes as a dark brown solid.

### Step 2: 3-bromo-2,6-dimethylimidazo[1,2-b]pyridazine

To a stirred solution of *2,6-dimethylimidazo[1,2-b]pyridazine* (36 g, 244.5 mmol) in acetonitrile (360 mL) was added N-bromosuccinimide (NBS) (52.2 g, 293.4 mmol) followed by stirring at ambient temperature for 1h. Upon completion, the acetonitrile in the reaction was distilled out. The obtained crude product was purified by flash column chromatography (neutral alumina) eluting the required compound 3-bromo-2,6-dimethylimidazo[1,2-b]pyridazine (18 g, 32.5%) with 15% ethyl acetate-hexanes as a solid.

### Step 3: 3-(3,4-dimethoxyphenyl)-2,6-dimethylimidazo[1,2-b]pyridazine

A 100 ml round bottom flask was charged with 3-bromo-2,6-dimethylimidazo[1,2-b]pyridazine (5 g, 17.6 mmol), boronic ester (4.4 g, 19.4 mmol), potassium carbonate (7.5 g, 54.3 mmol) and dioxane : water (45 ml : 5 ml). This solution was degassed with N₂ for 10 min and then the [1,1'-bis(diphenylphosphino)ferrocene]dichloropalladium(II). dichloromethane complex (Pd(dppf)Cl₂.DCM complex) (1.8 g, 2.2 mmol) was added. The reaction was heated at 100 °C for 16h. Upon completion, the reaction was diluted with ethyl acetate and filtered through a celite bed. The filtrate was partitioned between ethyl acetate and water. The ethyl acetate layer was dried over Na₂SO₄ and concentrated under reduced pressure. The obtained crude product was purified by flash column chromatography (neutral alumina) eluting the required compound 3-(3,4-dimethoxyphenyl)-2,6-dimethylimidazo[1,2-b]pyridazine (4.2 g, 67.7%) with 30% ethyl acetate-hexanes as an off-white solid.

### Step 4: 3-(3,4-dimethoxyphenyl)-8-iodo-2,6-dimethylimidazo[1,2-b]pyridazine

To a stirred solution of 2M lithium diisopropylamide (LDA) (0.7ml, 1.4 mmol) in THF at -78°C was added a solution of 3-(3,4-dimethoxyphenyl)-2,6-dimethylimidazo[1,2-b]pyridazine (0.2 g, 0.705 mmol) dissolved in THF (SmL), dropwise. After 10 min, iodine (0.178 g, 0.705 mmol) dissolved in THF (3mL) was added and the reaction was stirred at ambient temperature for 1h. Upon completion, the reaction was quenched with saturated NH₄Cl solution and extracted with ethyl acetate. The ethyl acetate layers were dried under Na₂SO₄ and concentrated under reduced pressure. The obtained crude product was purified by flash column chromatography (neutral alumina) eluting the required compound 3-(3,4-dimethoxyphenyl)-8-iodo-2,6-dimethylimidazo[1,2-b]pyridazine (30 mg, 10.7%) with 30% ethyl acetate-hexanes as a pale yellow solid.

### Example 1

### 3-(3,4-dimethoxyphenyl)-2,6-dimethyl-N-(pyridin-4-ylmethyl)imidazo [1,2-b] pyridazin-8-amine

### Step 1: Synthesis of 2,6-dimethylimidazo[1,2-b]pyridazine

A 3 neck 1L round bottom flask was charged with 6-methylpyridazin-3-amine (20 g, 183 mmol, 1 eq), ethanol (200 mL), and chloroacetone (24.4 g, 21.22 mL, 256 mmol, 1.4 eq). The reaction mixture was heated at 70 °C overnight. NaHCO3 (23.2 g, 276 mmol, 1.5 eq) was added portion wise. After most of bubbling subsides, the reaction was heated at 100 °C overnight. The solvents were removed in vacuo and the residue was taken up in dichloromethane and filtered through a filter paper. The solvent was again removed in vacuo. The residue was purified using silica gel chromatography with a hexanes and ethyl acetate gradient to obtain 2,6-dimethylimidazo[1,2-b]pyridazine (16.0 g, 59%).

### Step 2: Synthesis of 3-bromo-2,6-dimethylimidazo[1,2-b]pyridazine

To a solution of 2,6-dimethylimidazo[1,2-b]pyridazine (16.0 g, 10.8 mmol, 1.0 eq) in acetonitrile (320 mL, 30 V) was added N bromo succinamide (23.24 g, 130.6 mmol, 1.2 eq) under room temperature. The reaction mass was stirred at room temperature for 1 hour. Reaction was monitored by TLC. The crude reaction mass obtained upon evaporation of the volatiles were purified by silica gel (60-120) column chromatography (3% methanol in dichloromethane) to obtain 3-bromo-2,6-dimethylimidazo[1,2-b]pyridazine (15.0 g, 62%) as pale yellow solid.

### Step 3: Synthesis of 3-(3,4-dimethoxyphenyl)-2,6-dimethylimidazo[1,2-b]pyridazine

To a stirred solution of 3-bromo-2,6-dimethylimidazo[1,2-b]pyridazine (15.0 g, 66.3 mmol, 1.0 eq) and 3,4- dimethoxy phenyl boronic acid (14.5 g, 79.6 mmol, 1.2 eq) in dioxane: water (450 mL, 30V) were added potassium carbonate (22.9 g, 0.1659 mol, 2.5 eq) and PdCl₂(dppf)DCM (4.85 g, 6.6 mmol, 0.1eq) under room temperature. The resulting reaction mass was stirred at 115 °C over a period of 16 hours. Reaction was monitored by TLC. The reaction mass was diluted with ethyl acetate (600 mL) and filtered through celite pad. The organic layer was washed with water (400 mL), brine (400 mL), dried over anhydrous sodium sulphate and concentrated. The crude product obtained was purified by silica gel (230-400) column chromatography (6% methanol in dichloromethane) to obtain 3-(3,4-dimethoxyphenyl)-2,6-dimethylimidazo[1,2-b]pyridazine (10.0 g, 55%).

### Step 4: Synthesis of 3-(3,4-dimethoxyphenyl)-8-iodo-2,6-dimethylimidazo[1,2-b]pyridazine

To a stirred solution of 3-(3,4-dimethoxyphenyl)-2,6-dimethylimidazo[1,2-b]pyridazine (10.0 g, 35.3 mmol, 1.0 eq) in anhydrous THF (100 mL) under argon, at -70 °C, was added dropwise a solution of lithium diisopropylamide (2.0 M solution in THF, 17.64 mL, 35.3 mmol, 1.0 eq) followed by iodine (8.96 g, 35.3 mmol, 1.0 eq) in THF (100 mL, 10 V). The resulting reaction mass was stirred at same temperature for 1 h. Reaction was monitored by TLC. The reaction mass was quenched with saturated ammonium chloride solution (300 mL, 30 V) and extracted with ethyl acetate (2 x 500 mL). The combined organic layers were washed with brine, dried over anhydrous Na2SO4 and concentrated. The crude product obtained upon evaporation of the volatiles was purified by silica gel (230-400) column chromatography (3% methanol in dichloromethane) to obtain 3-(3,4-dimethoxyphenyl)-8-iodo-2,6-dimethylimidazo[1,2-b]pyridazine (6.5 g, 45%).

### Step 5: Synthesis of 3-(3,4-dimethoxyphenyl)-2,6-dimethyl-N-(pyridin-4-ylmethyl)imidazo[1,2-b]pyridazin-8-amine

To a solution of 3-(3,4-dimethoxyphenyl)-8-iodo-2,6-dimethylimidazo[1,2-b]pyridazine (7.5 g, 18.3 mmol, 1.0 eq)) and pyridin-4-ylmethanamine (2.57 g, 23.8 mmol, 1.3 eq) in toluene (225 mL, 30 V) under argon atmosphere were added cesium carbonate (11.94 g, 36.6 mmol, 2.0 eq) and BINAP (0.57 g, 0.91 mmol, 0.05 eq) at room temperature. The resulting reaction mass was degassed for 5 minutes, added palladium acetate (0.2 g, 0.91 mmol, 0.05 eq) and continued the stirring at 100 °C for a period of 16 hours. The resulting reaction mass was diluted with ethyl acetate (500 mL) and filtered through celite pad. Ethyl acetate layer was washed with water (300 mL) and brine (300 mL). Organic layer was dried over anhydrous sodium sulphate, filtered and concentrated under reduced pressure. The crude product obtained was purified by silica gel (230-400) column chromatography (3% methanol in dichloromethane) to obtain Example 1, yield 3.5 g, 49%, as an off-white solid.

### Example 2

### 3-(3,4-dimethoxyphenyl)-2,6-dimethyl-N-((2-methylpyridin-4-yl)methyl)imidazo[1,2-b]pyridazin-8-amine

To a stirred solution of 3-(3,4-dimethoxyphenyl)-8-iodo-2,6-dimethylimidazo[1,2-b]pyridazine (0.1 g, 0.240 mmol) and 1-(2-methylpyridin-4-yl)methanamine (0.038 g, 0.312 mmol) in toluene (2 mL) were added cesium carbonate (0.156 g, 0.48 mmol), 2,2'-bis(diphenylphosphino)-1,1'-binaphthyl (BINAP (7 mg, 0.012 mmol) and Pd(OAc)₂ (2 mg, 0.012 mmol). The reaction was stirred at 105 °C for 16h. Upon completion, the reaction was diluted with 10% MeOH-CH₂Cl₂ and filtered through a celite bed. The filtrate was concentrated and the obtained solid was washed with acetonitrile to afford Example 2 (0.09 g, 91.83%) as a pale brown solid.

### Example 3

### 3-(3,4-dimethoxyphenyl)-N-((2-fluoropyridin-4-yl)methyl)-2,6-dimethylimidazo [1,2-b]pyridazin-8-amine

To a stirred solution of 3-(3,4-dimethoxyphenyl)-8-iodo-2,6-dimethylimidazo[1,2-b]pyridazine (0.15 g, 0.360 mmol) and 1-(2-fluoropyridin-4-yl)methanamine (0.056 g, 0.46 mmol) in toluene (3 mL) were added cesium carbonate (0.23 g, 0.72 mmol), 2,2'-bis(diphenylphosphino)-1,1'-binaphthyl (BINAP) (11 mg, 0.018 mmol) and Pd(OAc)₂ (4 mg, 0.018 mmol). The reaction was stirred at 105°C for 16h. Upon completion, the reaction was diluted with 10% MeOH-CH₂Cl₂ and filtered through a celite bed. The filtrate was concentrated and the obtained solid was washed with acetonitrile to afford Example 3 (0.120 g, 80%) as a pale brown solid.

### Synthesis of 7-chloro-3-iodo-2,5-dimethylpyrazolo[1,5-a]pyrimidine

The synthetic intermediary 7-chloro-3-iodo-2,5-dimethylpyrazolo[1,5-a]pyrimidine was synthesized in a multistep process as follows:

### Step 1: 2,5-dimethylpyrazolo[1,5-a]pyrimidin-7-ol

A round bottom flask was charged with 3-amino-5-methylpyrazole (100 g, 1.02 mol), ethyl acetoacetate (161mL, 1.23 mol), acetic acid (300 mL) and 1,4-dioxane (1000 mL). The reaction was refluxed for 16h at 105°C. Off-white solids were obtained upon completion of the reaction and were filtered through suction. The solids were washed with cold hexane and dried under vacuum to obtain 2,5-dimethylpyrazolo[1,5-a]pyrimidin-7-ol (85 g, 49%) as an off-white solid.

### Step 2: 7-chloro-2,5-dimethylpyrazolo[1,5-a]pyrimidine

To a solution of 2,5-dimethylpyrazolo[1,5-a]pyrimidin-7-ol (120 g, 0.73 mol) in acetonitrile (1200 mL) was added POCl₃ (103 ml, 1.1 mol) dropwise. Upon completion of addition, the reaction was heated at 80°C for 12h. Upon completion, the POCl₃ in the reaction was distilled out. The crude product was diluted with water and neutralized with saturated NaHCOs solution and extracted with ethyl acetate. The organic layer was dried over sodium sulfate and concentrated under reduced pressure. The crude product was purified by flash column chromatography eluting the 7-chloro-2,5-dimethylpyrazolo[1,5-a]pyrimidine with 20% ethyl acetate-hexanes as an off-white solid (108 g, 80.8%).

### Step 3: 7-chloro-3-iodo-2,5-dimethylpyrazolo[1,5-a]pyrimidine

To an ice cold solution of 7-chloro-2,5-dimethylpyrazolo[1,5-a]pyrimidine (120 g, 0.66 mol) in acetonitrile (1200 mL) at -10°C was added N-iodosuccinimide (163.5 g, 0.726 mol) portion wise. The reaction was stirred at this temperature for 1h. Upon completion, solids were observed. The reaction was quenched with ice cold water and filtered via suction. The obtained solids were washed with hexane and dried under vacuum to afford 7-chloro-3-iodo-2,5-dimethylpyrazolo[1,5-a]pyrimidine as a white solid (182.8 g, 89.9%).

### Synthesis of 1,3-dimethyl-5-(4,4,5,5-tetramethyl-[1,3,2]dioxaborolan-2-yl)-1H-indazol

The boronic ester 1,3-dimethyl-5-(4,4,5,5-tetramethyl-[1,3,2]dioxaborolan-2-yl)-1H-indazol was synthesized in a multistep process as follows:

### Step 1: 5-bromo-1,3-dimethyl-1H-indazole

A stirred solution of 1-(5-bromo-2-fluorophenyl)ethanone (50 g, 230 mmol) and N-methyl hydrazine (42.4 mL, 805 mmol) in pyridine (500 mL) was heated at 90°C for 10h. Upon completion, the pyridine in the reaction was distilled out. The crude product was partitioned between water and ethyl acetate. The ethyl acetate layers were dried over sodium sulfate and concentrated. The obtained crude product was purified by flash column chromatography (neutral alumina) eluting the required compound 5-bromo-1,3-dimethyl-1H-indazole (20.23 g, 41.8%) with 2% ethyl acetate-hexanes as a pale brown viscous compound.

### Step 2: 1,3-dimethyl-5-(4,4,5,5-tetramethyl-[1,3,2]dioxaborolan-2-yl)-1H-indazole

To a solution of 5-bromo-1,3-dimethyl-1H-indazole (26 g, 115 mmol) and bispinacolato diboron (32.3 g, 127 mmol) in 1,4-dioxane (260 mL) was added KOAc (34 g, 345 mmol ). The reaction was degassed with N₂ for 10 min and then Pd(PPh₃)₄ (6.6 g, 5.57 mmol) was added and heated at 95°C for 16h. Upon completion of addition, the reaction was heated at 80°C for 12h. Upon completion, the reaction was filtered through celite, the filtrate was concentrated. The obtained crude product was purified by flash column chromatography (neutral alumina) eluting the required compound 1,3-dimethyl-5-(4,4,5,5-tetramethyl-[1,3,2]dioxaborolan-2-yl)-1H-indazole (18 g, 52.1%) with 5% ethyl acetate-hexanes as an off-white solid compound.

### Example 4

### 3-(1,3-dimethyl-1H-indazol-5-yl)-2,5-dimethyl-N-[(2-fluoropyridin-4-yl)methyl]pyrazolo[1,5-a]pyrimidin-7-amine

### Step 1: N-[(2-fluoropyridin-4-yl)methyl]-3-iodo-2,5-dimethylpyrazolo[1,5-a]pyrimidin-7-amine

To a stirred solution of 7-chloro-3-iodo-2,5-dimethylpyrazolo[1,5-a]pyrimidine (1.1 g, 3.57 mmol) and 1-(2-fluoropyridin-4-yl)methanamine (0.586 g, 4.6 mmol) in ethanol (5.5 mL) was added diisopropylethylamine (5.5 mL, 5 Vols) and stirred at 80°C for 6h. Upon completion, the ethanol in the reaction was distilled out. The crude product was partitioned between water and ethyl acetate. The ethyl acetate layers were dried over sodium sulfate and concentrated under reduced pressure. Flash column chromatography eluted the required compound with 35% ethyl acetate-hexane. Upon concentration it afforded N-[(2-fluoropyridin-4-yl)methyl]-3-iodo-2,5-dimethylpyrazolo[1,5-a]pyrimidin-7-amine (0.9 g, 63.82%) as a pale yellow compound.

### Step 2: tert-butyl [(2-fluoropyridin-4yl)methyl](3-iodo-2,5-dimethylpyrazolo[1,5-a]pyrimidin-7-yl)carbamate

To a stirred solution of N-[(2-fluoropyridin-4-yl)methyl]-3-iodo-2,5-dimethylpyrazolo[1,5-a]pyrimidin-7-amine (0.5 g, 1.26 mmol) in dichloromethane (DCM) (SmL) was added 4-dimethylaminopyridine (DMAP) (0.153 g, 1.26 mmol) at 0°C. Then Boc anhydride (di-*tert-*butyl dicarbonate) (0.33 ml, 1.51 mmol) was added dropwise at this temperature and stirred at ambient temperature for 2h. Upon completion, the reaction mixture was washed with water. The organic layer then was dried over Na₂SO₄ and concentrated. The obtained crude product was purified by flash column chromatography (neutral alumina) eluting the required compound tert-butyl [(2-fluoropyridin-4-yl)methyl](3-iodo-2,5-dimethylpyrazolo[1,5-a]pyrimidin-7-yl)carbamate (0.51 g, 82.2% ) with 10% ethyl acetate-hexanes as an off-white solid.

### Step 3: [3-(1,3-Dimethyl-1H-indazol-5-yl)-2,5-dimethyl-pyrazolo[1,5-a]pyrimidin-7-yl]-(2-fluoro-pyridin-4-ylmethyl)-carbamic acid tert-butyl ester

A 25 ml round bottom flask was charged with tert-butyl [(2-fluoropyridin-4-yl)methyl](3-iodo-2,5-dimethylpyrazolo[1,5-a]pyrimidin-7-yl)carbamate (0.45 g, 0.904 mmol), 1,3-dimethyl-5-(4,4,5,5-tetramethyl-[1,3,2]dioxaborolan-2-yl)-1H-indazole (0.322g, 1.17mmol), potassium carbonate (0.311g, 2.26mmol) and dioxane : water (4.5 mL:0.5 mL). This solution was degassed with N₂ for 10 min and then the Pd(dppf)Cl₂.DCM complex (0.110 g, 0.135 mmol) was added. The reaction was heated at 100°C for 16h. Upon completion, the reaction was diluted with ethyl acetate and filtered through a celite bed. The filtrate was partitioned between ethyl acetate and water. The ethyl acetate layer was dried over Na₂SO₄ and concentrated under reduced pressure. The obtained crude product was purified by flash column chromatography (neutral alumina) eluting the required compound [3-(1,3-Dimethyl-1H-indazol-5-yl)-2,5-dimethyl-pyrazolo[1,5-a]pyrimidin-7-yl]-(2-fluoro-pyridin-4-ylmethyl)-carbamic acid tert-butyl ester (0.2 g, 42.9%) with 30% ethyl acetate-hexanes as an off-white solid.

### Step 4: 3-(1,3-dimethyl-1H-indazol-5-yl)-2,5-dimethyl-N-[(2-fluoropyridin-4-yl)methyl]pyrazolo[1,5-a]pyrimidin-7-amine

To an ice cold solution of [3-(1,3-Dimethyl-1H-indazol-5-yl)-2,5-dimethyl-pyrazolo[1,5-a]pyrimidin-7-yl]-(2-fluoro-pyridin-4-ylmethyl)-carbamic acid tert-butyl ester (0.2 g, 0.388 mmol) in dichloromethane (2mL) was added trifluoroacetic acid (2mL) dropwise. After completion of the addition, the reaction mass was stirred at ambient temperature for 6 hours. After completion of the reaction, the solvent and excess trifluoroacetic acid were distilled out. The crude product was basified with 1N sodium hydroxide solution and extracted with 10% methanol-dichloromethane. The organic layers were dried over sodium sulfate and concentrated. The obtained crude product was purified by flash column chromatography (neutral alumina) eluting the required compound, Example 4 (0.06 g, 37.5%), with 70% ethyl acetate-hexanes as an off-white solid.

### Example 5

### 3-(1,3-dimethyl-1H-indazol-5-yl)-2,5-dimethyl-N-[(2-methylpyridin-4-yl)methyl]pyrazolo[1,5-a]pyrimidin-7-amine

### Step 1: 3-iodo-2,5-dimethyl-N-[(2-methylpyridin-4-yl)methyl]pyrazolo[1,5-a]pyrimidin-7-amine

To a stirred solution of 7-chloro-3-iodo-2,5-dimethylpyrazolo[1,5-a]pyrimidine (1.1 g, 3.57 mmol) and 1-(2-methylpyridin-4-yl)methanamine (0.586 g, 4.6 mmol) in ethanol (5.5 ml) was added diisopropylethylamine (5.5 mL, 5 Vols) and the reaction mixture was then stirred at a temperature of 80°C for 6 hours. Upon completion, the ethanol in the reaction was distilled out. The crude reaction product was partitioned between water and ethyl acetate. The ethyl acetate layers were dried over sodium sulfate and concentrated under reduced pressure. Flash column chromatography was performed to elute the required compound with 35 % ethyl acetate-hexane. Concentration yielded 3-iodo-2,5-dimethyl-N-[(2-methylpyridin-4-yl)methyl]pyrazolo[1,5-a]pyrimidin-7-amine (0.9 g, 40.1%) as a pale yellow compound.

### Step 2: (3-iodo-2,5-dimethyl-pyrazolo[1,5-a]pyrimidin-7-yl)-(2-methyl-pyridin-4-ylmethyl)-carbamic acid tert-butyl ester

To a stirred solution of 3-iodo-2,5-dimethyl-N-[(2-methylpyridin-4-yl)methyl]pyrazolo[1,5-a]pyrimidin-7-amine (0.5 g, 1.26 mmol) in dichloromethane (5mL) was added DMAP (0.153 g, 1.26 mmol) at 0°C. Then Boc anhydride (0.33 ml, 1.51 mmol) was added dropwise at this temperature and stirred at ambient temperature for 2 hours. Upon completion of reaction, the reaction mixture was washed with water. The organic layer then was dried over sodium sulfate and concentrated. The obtained crude product was purified by flash column chromatography (neutral alumina) eluting the required compound (3-iodo-2,5-dimethyl-pyrazolo[1,5-a]pyrimidin-7-yl)-(2-methyl-pyridin-4-ylmethyl)-carbamic acid tert-butyl ester (0.51 g, 82.2 %) with 10% ethyl acetate-hexanes as an off-white solid.

### Step 3: [3-(1, 3-Dimethyl-1H-indazol-5-yl)-2, 5-dimethyl-pyrazolo[1,5-a]pyrimidin-7-yl]-(2-methyl-pyridin-4-ylmethyl)-carbamic acid tert-butyl ester

A 25 ml round bottom flask was charged with (3-iodo-2,5-dimethyl-pyrazolo[1,5-a]pyrimidin-7-yl)-(2-methyl-pyridin-4-ylmethyl)-carbamic acid tert-butyl ester (0.45 g, 0.904 mmol), 1,3-dimethyl-5-(4,4,5,5-tetramethyl-[1,3,2]dioxaborolan-2-yl)-1H-indazole (0.322 g, 1.17 mmol), potassium carbonate (0.311 g, 2.26 mmol) and dioxane : water (4.5 mL : 0.5 mL). This solution was degassed with N₂ for 10 min and then the Pd(dppf)Cl₂.dichloromethane complex (0.110 g, 0.135 mmol) was added. The reaction was heated at 100°C for 16 hours. Upon completion of the heating, the reaction was diluted with ethyl acetate and was filtered through a celite bed. The filtrate then was partitioned between ethyl acetate and water. The ethyl acetate layer was dried over sodium sulfate and was concentrated under reduced pressure. The obtained crude product was purified by flash column chromatography (neutral alumina) eluting the required compound [3-(1,3-dimethyl-1H-indazol-5-yl)-2,5-dimethyl-pyrazolo[1, 5-a]pyrimidin-7-yl]-(2-methyl-pyridin-4-ylmethyl)-carbamic acid tert-butyl ester (0.2 g, 43.4%) with 30 % ethyl acetate-hexanes as an off-white solid.

### Step 4: 3-(1,3-dimethyl-1H-indazol-5-yl)-2,5-dimethyl-N-[(2-methylpyridin-4-yl)methyl]pyrazolo[1,5-a]pyrimidin-7-amine

To an ice cold solution of [3-(1,3-Dimethyl-1H-indazol-5-yl)-2,5-dimethyl-pyrazolo[1,5-a]pyrimidin-7-yl]-(2-methyl-pyridin-4-ylmethyl)-carbamic acid tert-butyl ester (0.2 g, 0.388 mmol) in dichloromethane (2 mL) was added trifluoroacetic acid (TFA) (2 mL) dropwise. After completion of the addition, the reaction mass was stirred at ambient temperature for 6 hours. After completion of the reaction, the solvent and excess TFA were distilled out.

The crude product was basified with 1N NaOH solution and was extracted with 10% MeOH-DCM. The organic layers were dried over Na₂SO₄ and then were concentrated. The obtained crude product was purified by flash column chromatography (neutral alumina) eluting the required compound Example 5 (0.06 g, 37.7%) with 70% ethyl acetate-hexanes as an off-white solid.

### Examples 6-9

Examples 6-9 were synthesized using the same general methods as described for Examples 4 and 5. Analytical data for the compounds of Examples 1-9 are shown in Table 2.

**Table 2**

| **Example No.** | **Analytical Data** |
|---|---|
| 1 | ¹H-NMR (DMSO₆, 400 MHz): δ 8.50 (d, 2H), 87.97 (s, 1H), 7.35 (d, 2H), 7.28 (s, 1H), 7.17 (d, 1H), 7.07 (d, 1H), 5.97 (s, 1H), 4.57 (d, 2H), 3.80. (d, 6H), 2.49 (s, 3H), 2.23 (s, 3H), LCMS: 390.2[M+H], HPLC purity: 98.46% |
| 2 | ¹H-NMR (DMSO-d6, 300 MHz): δ 8.36 (d, 1 H), 7.95 (s, 1 H), 7.28 (d, 1 H), 7.16 (m, 3 H), 7.07 (d, 1H), 5.82 (s, 1 H), 4.15 (s, 2 H), 3.78 (d, 6 H), 2.43 (s, 3 H), 2.23 (s, 3 H), LCMS : 404.3 [M+H], HPLC purity: 99.9% |
| 3 | ¹H-NMR (DMSO-d6, 300 MHz): δ 8.18 (d, 1 H), 8.02 (s, 1 H), 7.33 (d, 1H), 7.28 (d, 1 H), 7.16 (dd, 1 H), 7.1 (d, 1H), 7.07 (d, 1H), 5.91 (s, 1 H), 4.61 (s, 2 H), 3.80 (d, 6 H), 2.43 (s, 3 H), 2.24 (s, 3 H), LCMS : 408.5 [M+H], HPLC purity: 99.5% |
| 4 | ¹H-NMR (CDCl₃, 300 MHz): δ 8.25 (d, 1 H), 7.88 (s, 1 H), 7.75 (dd, 1H), 7.42 (d, 1 H), 7.22 (d, 1 H), 6.97 (s, 1H), 6.76 (dd, 1H), 5.68 (s, 1 H), 4.70 (s, 2 H), 4.03 (d, 3 H), 2.61 (s, 6 H), 2.47 (s, 3 H), LCMS : 416.4 [M+H], HPLC purity: 99.7% |
| 5 | ¹H-NMR (CDCl₃, 300 MHz): δ 8.52 (d, 1 H), 7.88 (s, 1 H), 7.75 (dd, 1H), 7.41 (d, 1 H), 7.21 (d, 1 H), 7.17 (d, 1H), 6.73 (m, 1H), 5.69 (s, 1 H), 4.64 (s, 2 H), 4.03 (d, 3 H), 2.61 (s, 9 H), 2.47 (s, 3 H), LCMS : 412.3 [M+H], HPLC purity: 99.6% |
| 6 | ¹H-NMR (CDCl₃, 300 MHz): δ 8.52 (d, 1 H), 7.88 (s, 1 H), 7.75 (dd, 1H), 7.42 (d, 1 H), 7.18 (s, 1 H), 7.14 (d, 1H), 6.69 (m, 1H), 5.71 (s, 1 H), 4.63 (s, 2 H), 4.03 (d, 3 H), 2.85 (q, 2 H), 2.61 (s, 6 H), 2.47 (s, 3 H), 1.32 (t, 3 H), LCMS : 426.4 [M+H], HPLC purity: 99.7% |
| 7 | ¹H-NMR (CDCl₃, 300 MHz): δ 8.18 (d, 1 H), 7.88 (s, 1 H), 7.75 (dd, 1H), 7.41 (d, 1 H), 6.89 (d, 1H), 6.73 (m, 2H), 5.72 (s, 1 H), 4.60 (s, 2 H), 4.03 (d, 3 H), 3.95 (s, 3 H), 2.60 (s, 6 H), 2.48 (s, 3 H), LCMS : 428.4 [M+H], HPLC purity: 99.5% |
| 8 | ¹H-NMR (CDCl₃, 300 MHz): δ 8.59 (d, 1 H), 8.25 (s, 1 H), 7.81 (m, 2H), 7.72 (d, 1 H), 7.49 (d, 1H), 7.41 (d, 1H), 5.68 (s, 1 H), 4.75 (d, 2 H), 4.03 (d, 3 H), 2.60 (s, 6 H), 2.48 (s, 3 H), LCMS : 441.5 [M+18], HPLC purity: 93.7% |
| 9 | ¹H-NMR (CDCl₃, 300 MHz): δ 8.76 (d, 1 H), 7,88 (s, 1 H), 7.75 (m, 2H), 7.53 (d, 1 H), 7.42 (d, 1H), 6.80 (s, 1H), 5.68 (s, 1 H), 4.75 (d, 2 H), 4.03 (d, 3 H), 2.60 (s, 6 H), 2.48 (s, 3 H), LCMS : 466.4 [M+18], HPLC purity: 99.35% |

### Liposomal formulation

50 mg of 3-(1,3-dimethyl-1H-indazol-5-yl)-2,5-dimethyl-N-[(2-fluoropyridin-4-yl)methyl]pyrazolo[1,5-a]pyrimidin-7-amine (Example 4) and 500 mg of soybean lecithin were transferred to a beaker. Chloroform (about 10 ml) was added and the mixture was kept under stirring until the components had dissolved completely. Then the solvent was removed by rotary evaporator to yield in a thin lipid film. Water (10 ml) was added to the lipid film and the film was allowed to rehydrate at room temperature. The formulation was mixed thoroughly and sonicated to form a fine dispersion. The formulation had a pH of 6.42.

### Biological Assays

### In vitro study of antiviral activity and cell toxicity

The antiviral activity of compounds of the invention has been evaluated at a concentration range of 0.032 to 20 µM for anti-RSV activity by a standard plaque reduction assay (Lundin et al, Antiviral Methods and protocols, 2013 Ed. 2, vol. 1030, page 345-364). Human laryngeal epidermoid carcinoma (HEp-2) cells and RSV strain A2 (ATCC, VR-1540) were used for the evaluation. The cell viability assay using tetrazolium salt (MTS) was also performed in HEp-2 cells. The IC₅₀ and CC₅₀ found for tested compounds are shown in Table 3, together with the calculated selectivity index (SI).

**Table 3**

| **Tested Compound** | **IC₅₀ (RSV) (nM)** | **CC₅₀ (nM)** | **SI (CC₅₀/IC₅₀)** |
|---|---|---|---|
| PIK-93 | 400 | 40000 | 100 |
| Pleconaril | 8000 | 300000 | 38 |
| Vapendavir | >20000 | 300000 | <15 |
| Example 1 | 30 | 60000 | 2000 |
| Example 5 | 40 | 18000 | 450 |
| Example 6 | 50 | 160000 | 3200 |
| Example 25 | 20 | >500000 | >25000 |
| Example 29 | 40 | 60000 | 150 |
| Example 38 | 90 | 60000 | 667 |
| Example 45 | 70 | >500000 | >7143 |
| Example 61 | 34 | 18000 | 514 |
| Example 68 | 40 | 40000 | 1000 |

### In vitro study of inhibition of extracellular and cell-associated virus

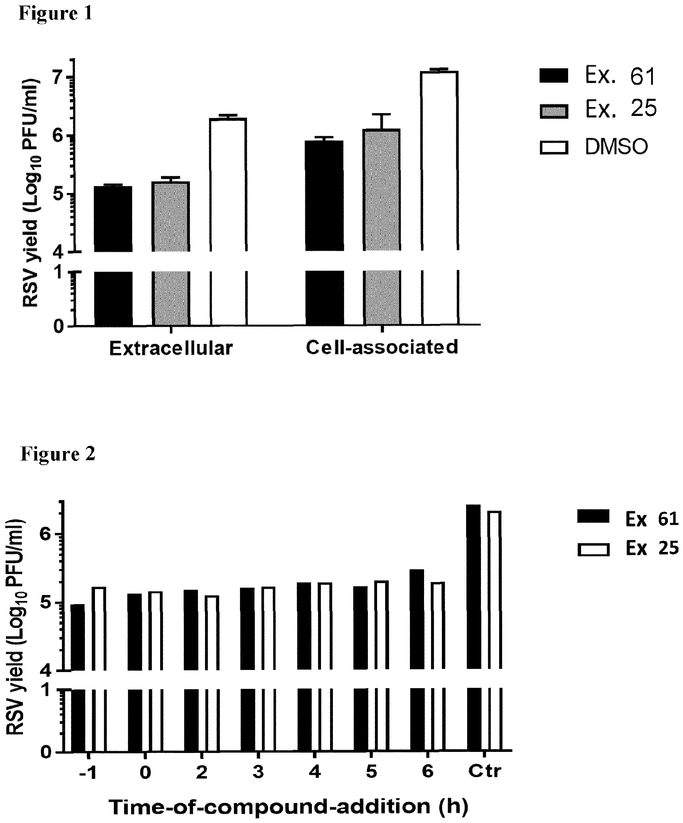

The titer of extracellular (EX) and cell-associated (CA) RSV after incubation in Hep-2 cells at a multiplicity of infection (MOI) of 3 was determined by a plaque titration assay. Infected cells were harvested by scraping into fresh supernatant medium and then subjected to a rapid freeze-thaw cycle at -80 °C ethanol and a 37 °C water bath, respectively, to release infectious virus. The results are illustrated in **Figure 1**, which shows the inhibition of (EX) and c(CA) RSV yield in HEp-2 cells by the compounds of Examples 25 and 61, tested at 0.2 µM. Both compounds inhibited RSV yield of EX and CA virus by approximately 1 log10.

### In vitro study of time dependency of antiviral effect

A time-of-addition study was performed using the compounds of Examples 25 and 61. Both compounds (at 0.2 µM) were added at different time points (from -1h through 0h (beginning of infection), 2h (end of infection), 3h (1h after infection), 4h, 5h, and 6h relative to RSV infected HEp-2 cells. At these conditions both compounds showed time-independent inhibition of RSV yield, suggesting that the compounds affect a step of viral life cycle occurring at >6h after infection. The results are illustrated in **Figure 2****.**

### Plasma and lung tissue pharmacokinetic study

### Formulation Vehicle: Liposomal formulation (Soybean lecithin (Phosphotidyl Choline)

*Step 1*: Exactly 225 mg of the compound of Example 68 and 2250 mg of soya lecithin were transferred to a beaker. About 30 mL of Chloroform were added and kept for stirring until all the components were dissolved completely.

*Step 2:* Solvent was removed by rotary evaporator to yield in a thin lipid film.

*Step 3:* 45 mL of water were added to the lipid film and kept for swelling at room temperature. The formulation was mixed well and sonicated to form a fine dispersion. The pH of the formulation was 6.67

### Study Procedure: Single Dose Oral PK and TD Study

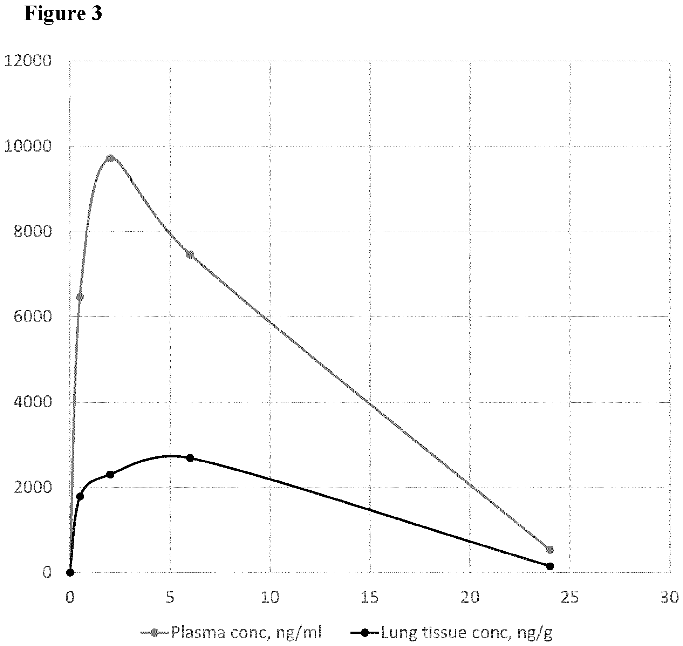

*Dose Administration*: Adult healthy male Sprague Dawley rats aged 8-10 weeks were used for experimentation after a minimum 3 days of acclimation. Fed state animals were administered with the compound of Example 68 in recommended vehicle (Liposomal formulation - Soybean lecithin) by oral route with a dose of 50 mg/kg body weight and at dose volume of 10 mL/kg body weight. Under mild isoflurane anesthesia, blood specimens were collected into pre-labeled tubes containing anticoagulant (K₂EDTA - 2 mg/mL blood) during the next 24 hours of post-dose. Collected blood specimens were centrifuged at 4000 rpm, 4°C for 10 minutes, and plasma was separated and stored at -80°C until analysis. At 0.5, 2, 6 and 24 hours after blood collection, animals were euthanized and tissues (Pancreas, Lung, Heart, Intestine, Liver, Brain and Kidney) were collected. Collected tissue weights were taken and stored at -80°C until analysis. The results are illustrated in **Figure 3****,** showing the plasma and lung tissue concentration of compound Ex. 68 after administration of a 50 mg/kg dose per orally in Sprague Dawley rats. The results indicate that per oral treatment could be used for inhibition of RSV replication in the lungs as well in other systemic tissues.

## Claims

1. A compound of formula (I) 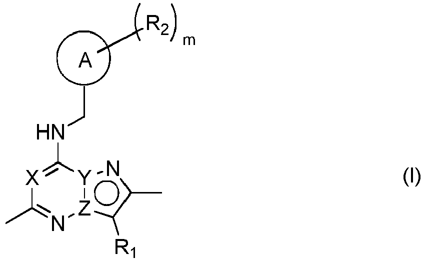 or a pharmaceutically acceptable salt thereof, wherein
X is CH, Y is C, and Z is N, or
X is CH, Y is N, and Z is C, or
X is N, Y is N, and Z is C;
R₁ is 3,4-dimethoxyphenyl or 1,3-dimethyl-1H-indazol-5-yl;
ring A is phenyl, or 5- or 6-membered heteroaryl;
m is 0, 1, 2 or 3;
each R₂ is independently selected from C1-C6 alkyl, halogen, R₃O, R₄R₅N, R₆R₇NS(O)₂, R₈R₉NC(O), R₁₀S(O)₂, R₁₁S(O)₂N(R₁₂), R₁₃C(O)O, R₁₄C(O)N(R₁₅), R₁₆OC(O), R₁₇C(O), R₁₈C(O)N(R₁₉)S(O)₂, R₂₀OC(O)N(R₂₁)S(O)₂, CN and -O⁻; and two R₃, attached to adjacent atoms of ring A, together with the atoms to which they are attached may form a 5- or 6-membered heterocyclic or carbocyclic ring;
each R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, and R₂₁ is independently selected from H and C1-C6 alkyl; and
any alkyl is optionally substituted by one or more F;
for use in the treatment of a *Pneumoviridae* viral infection.

2. The compound or pharmaceutically acceptable salt for use according to claim 1, wherein ring A is phenyl or 6-membered heteroaryl containing one or more nitrogen atoms in the ring.

3. The compound or pharmaceutically acceptable salt thereof for use according to claim 1 or 2, wherein ring A is phenyl or pyridinyl.

4. The compound or pharmaceutically acceptable salt for use according to any one of claims 1 to 3, wherein ring A is 4-pyridinyl.

5. The compound or pharmaceutically acceptable salt for use according to any one of claims 1 to 4, wherein X is CH.

6. The compound or pharmaceutically acceptable salt for use according to any one of claims 1 to 5, wherein Y is N and Z is C.

7. The compound or pharmaceutically acceptable salt for use according to any one of claims 1 to 6, wherein R₁ is 3,4-dimethoxyphenyl.

8. The compound or pharmaceutically acceptable salt for use according to any one of claims 1 to 6, wherein R₁ is 1,3-dimethyl-1H-indazol-5-yl.

9. The compound or pharmaceutically acceptable salt for use according to any one of claims 1 to 8, wherein m is 0 or 1.

10. The compound or pharmaceutically acceptable salt for use according to claim 1, wherein the compound is selected from
3-(3,4-dimethoxyphenyl)-2,6-dimethyl-N-(pyridin-4-ylmethyl)imidazo[1,2-b]pyridazin-8-amine,
3-(3,4-dimethoxyphenyl)-2,6-dimethyl-N-((2-methylpyridin-4-yl)methyl)imidazo[1,2-b]pyridazin-8-amine,
3-(3,4-dimethoxyphenyl)-N-((2-fluoropyridin-4-yl)methyl)-2,6-dimethylimidazo[1,2-b]pyridazin-8-amine,
3-(1,3-dimethyl-1H-indazol-5-yl)-2,5-dimethyl-N-[(2-fluoropyridin-4-yl)methyl]pyrazolo[1,5-a]pyrimidin-7-amine,
3-(1,3-dimethyl-1H-indazol-5-yl)-2,5-dimethyl-N-[(2-methylpyridin-4-yl)methyl]pyrazolo[1,5-a]pyrimidin-7-amine,
3-(1,3-dimethyl-1H-indazol-5-yl)-2,5-dimethyl-N-[(2-ethylpyridin-4-yl)methyl]pyrazolo[1,5-a]pyrimidin-7-amine,
3-(1,3-dimethyl-1H-indazol-5-yl)-2,5-dimethyl-N-[(2-methoxypyridin-4-yl)methyl]pyrazolo[1,5-a]pyrimidin-7-amine,
3-(1,3-dimethyl-1H-indazol-5-yl)-2,5-dimethyl-N-[(2-cyanopyridin-4-yl)methyl]pyrazolo[1,5-a]pyrimidin-7-amine,
3-(1,3-dimethyl-1H-indazol-5-yl)-2,5-dimethyl-N-[(2-trifluoromethylpyridin-4-yl)methyl]pyrazolo[1,5-a]pyrimidin-7-amine,
N-[(4-chlorophenyl)methyl]-3-(3,4-dimethoxyphenyl)-2,5-dimethyl-pyrazolo[1,5-a]pyrimidin-7-amine,
3-(3,4-dimethoxyphenyl)-N-[(4-methoxyphenyl)methyl]-2,5-dimethyl-pyrazolo[1,5-a]pyrimidin-7-amine,
3-(3,4-dimethoxyphenyl)-2,5-dimethyl-N-(p-tolylmethyl)pyrazolo[1,5-a]pyrimidin-7-amine,
N-(1,3-benzodioxol-5-ylmethyl)-3-(3,4-dimethoxyphenyl)-2,5-dimethyl-pyrazolo[1,5-a]pyrimidin-7-amine,
3-(3,4-dimethoxyphenyl)-N-[(4-fluorophenyl)methyl]-2,5-dimethyl-pyrazolo[1,5-a]pyrimidin-7-amine,
3-(3,4-dimethoxyphenyl)-2,5-dimethyl-N-(2-pyridylmethyl)pyrazolo[1,5-a]pyrimidin-7-amine,
N-benzyl-3-(3,4-dimethoxyphenyl)-2,5-dimethyl-pyrazolo[1,5-a]pyrimidin-7-amine,
3-(3,4-dimethoxyphenyl)-2,5-dimethyl-N-[[4-(trifluoromethyl)phenyl]methyl]pyrazolo[1,5-a]pyrimidin-7-amine,
3-(3,4-dimethoxyphenyl)-2,5-dimethyl-N-(4-pyridylmethyl)pyrazolo[1,5-a]pyrimidin-7-amine,
3-(3,4-dimethoxyphenyl)-2,5-dimethyl-N-[[4-(trifluoromethoxy)phenyl]methyl]pyrazolo[1,5-a]pyrimidin-7-amine,
N-[4-[[[3-(3,4-dimethoxyphenyl)-2,5-dimethyl-pyrazolo[1,5-a]pyrimidin-7-yl]amino]methyl]phenyl]acetamide,
3-(3,4-dimethoxyphenyl)-N-[(4-dimethylaminophenyl)methyl]-2,5-dimethyl-pyrazolo[1,5-a]pyrimidin-7-amine,
3-(3,4-dimethoxyphenyl)-N-[(6-methoxy-3-pyridyl)methyl]-2,5-dimethyl-pyrazolo[1,5-a]pyrimidin-7-amine,
N-[4-[[[3-(3,4-dimethoxyphenyl)-2,5-dimethyl-pyrazolo[1,5-a]pyrimidin-7-yl]amino]methyl]phenyl]methanesulfonamide,
4-[[[3-(3,4-dimethoxyphenyl)-2,5-dimethyl-pyrazolo[1,5-a]pyrimidin-7-yl]amino]methyl]phenol,
3-(3,4-dimethoxyphenyl)-2,5-dimethyl-N-[(3-methylsulfonylphenyl)methyl]pyrazolo[1,5-a]pyrimidin-7-amine,
3-(3,4-dimethoxyphenyl)-2,5-dimethyl-N-[(4-methylsulfonylphenyl)methyl]pyrazolo[1,5-a]pyrimidin-7-amine,
N-[(4-tert-butylphenyl)methyl]-3-(3,4-dimethoxyphenyl)-2,5-dimethyl-pyrazolo[1,5-a]pyrimidin-7-amine,
3-(3,4-dimethoxyphenyl)-2,5-dimethyl-N-[(2-methylpyrimidin-4-yl)methyl]pyrazolo[1,5-a]pyrimidin-7-amine,
3-(3,4-dimethoxyphenyl)-2,5-dimethyl-N-[(6-methyl-3-pyridyl)methyl]pyrazolo[1,5-a]pyrimidin-7-amine,
1-[4-[[[3-(3,4-dimethoxyphenyl)-2,5-dimethyl-pyrazolo[1,5-a]pyrimidin-7-yl]amino]methyl]phenyl]ethanone,
3-(3,4-dimethoxyphenyl)-2,5-dimethyl-N-(1-naphthylmethyl)pyrazolo[1,5-a]pyrimidin-7-amine,
4-[[[3-(3,4-dimethoxyphenyl)-2,5-dimethyl-pyrazolo[1,5-a]pyrimidin-7-yl]amino]methyl]benzenesulfonamide,
8-(3,4-dimethoxyphenyl)-N-[(4-fluorophenyl)methyl]-2,7-dimethyl-pyrazolo[1,5-a][1,3,5]triazin-4-amine,
8-(3,4-dimethoxyphenyl)-2,7-dimethyl-N-[[4-(trifluoromethyl)phenyl]methyl]pyrazolo[1,5-a][1,3,5]triazin-4-amine,
8-(3,4-dimethoxyphenyl)-2,7-dimethyl-N-(p-tolylmethyl)pyrazolo[1,5-a][1,3,5]triazin-4-amine,
8-(3,4-dimethoxyphenyl)-N-[(4-isopropylphenyl)methyl]-2,7-dimethyl-pyrazolo[1,5-a][1,3,5]triazin-4-amine,
8-(3,4-dimethoxyphenyl)-2,7-dimethyl-N-(2-pyridylmethyl)pyrazolo[1,5-a][1,3,5]triazin-4-amine,
8-(3,4-dimethoxyphenyl)-2,7-dimethyl-N-(4-pyridylmethyl)pyrazolo[1,5-a][1,3,5]triazin-4-amine,
N-[(4-chlorophenyl)methyl]-8-(3,4-dimethoxyphenyl)-2,7-dimethyl-pyrazolo[1,5-a][1,3,5]triazin-4-amine,
N-[4-[[[8-(3,4-dimethoxyphenyl)-2,7-dimethyl-pyrazolo[1,5-a][1,3,5]triazin-4-yl]amino]methyl]phenyl]acetamide,
8-(3,4-dimethoxyphenyl)-N-[(4-methoxyphenyl)methyl]-2,7-dimethyl-pyrazolo[1,5-a][1,3,5]triazin-4-amine,
8-(3,4-dimethoxyphenyl)-2,7-dimethyl-N-[(3-methylsulfonylphenyl)methyl]pyrazolo[1,5-a][1,3,5]triazin-4-amine,
8-(3,4-dimethoxyphenyl)-2,7-dimethyl-N-[(1-oxidopyridin-1-ium-4-yl)methyl]pyrazolo[1,5-a][1,3,5]triazin-4-amine,
8-(3,4-dimethoxyphenyl)-N-[(6-methoxy-3-pyridyl)methyl]-2,7-dimethyl-pyrazolo[1,5-a][1,3,5]triazin-4-amine,
4-[[[8-(3,4-dimethoxyphenyl)-2,7-dimethyl-pyrazolo[1,5-a][1,3,5]triazin-4-yl]amino]methyl]benzenesulfonamide,
N-(1,3-benzodioxol-5-ylmethyl)-8-(3,4-dimethoxyphenyl)-2,7-dimethyl-pyrazolo[1,5-a][1,3,5]triazin-4-amine,
4-[[[8-(3,4-dimethoxyphenyl)-2,7-dimethyl-pyrazolo[1,5-a][1,3,5]triazin-4-yl]amino]methyl]phenol,
N-[4-[[[8-(3,4-dimethoxyphenyl)-2,7-dimethyl-pyrazolo[1,5-a][1,3,5]triazin-4-yl]amino]methyl]phenyl]methanesulfonamide,
N-benzyl-8-(3,4-dimethoxyphenyl)-2,7-dimethyl-pyrazolo[1,5-a][1,3,5]triazin-4-amine,
8-(3,4-dimethoxyphenyl)-2,7-dimethyl-N-(2-thienylmethyl)pyrazolo[1,5-a][1,3,5]triazin-4-amine,
8-(3,4-dimethoxyphenyl)-2,7-dimethyl-N-(3-pyridylmethyl)pyrazolo[1,5-a][1,3,5]triazin-4-amine,
8-(3,4-dimethoxyphenyl)-2,7-dimethyl-N-[(5-methyl-2-furyl)methyl]pyrazolo[1,5-a][1,3,5]triazin-4-amine,
methyl N-[4-[[[8-(3,4-dimethoxyphenyl)-2,7-dimethyl-pyrazolo[1,5-a][1,3,5]triazin-4-yl]amino]methyl]phenyl]sulfonylcarbamate,
N-[4-[[[8-(3,4-dimethoxyphenyl)-2,7-dimethyl-pyrazolo[1,5-a][1,3,5]triazin-4-yl]amino]methyl]phenyl]sulfonylpropanamide,
N-methyl-4-[[[8-(3,4-dimethoxyphenyl)-2,7-dimethyl-pyrazolo[1,5-a][1,3,5]triazin-4-yl]amino]methyl]benzenesulfonamide,
8-(3,4-dimethoxyphenyl)-2,7-dimethyl-N-[(1-methyl-1H-pyrazol-3-yl)methyl]pyrazolo[1,5-a][1,3,5]triazin-4-amine,
8-(3,4-dimethoxyphenyl)-2,7-dimethyl-N-[(1,3-oxazol-5-yl)methyl]pyrazolo[1,5-a][1,3,5]triazin-4-amine,
8-(3,4-dimethoxyphenyl)-2,7-dimethyl-N-[(2-methylpyrimidin-5-yl)methyl]pyrazolo[1,5-a][1,3,5]triazin-4-amine,
8-(3,4-dimethoxyphenyl)-2,7-dimethyl-N-[(pyridazin-4-yl)methyl]pyrazolo[1,5-a][1,3,5]triazin-4-amine,
8-(3,4-dimethoxyphenyl)-2,7-dimethyl-N-[(6-methylpyridin-3-yl)methyl]pyrazolo[1,5-a][1,3,5]triazin-4-amine,
3-(1,3-dimethyl-1H-indazol-5-yl)-2,6-dimethyl-N-(pyridin-4-ylmethyl)imidazo[1,2-b]pyridazin-8-amine,
3-(1,3-dimethyl-1H-indazol-5-yl)-2,6-dimethyl-N-((6-methylpyridin-3-yl)methyl)imidazo[1,2-b]pyridazin-8-amine,
N-(4-chlorobenzyl)-3-(1,3-dimethyl-1H-indazol-5-yl)-2,6-dimethylimidazo[1,2-b]pyridazin-8-amine,
3-(1,3-dimethyl-1H-indazol-5-yl)-N-((6-methoxypyridin-3-yl)methyl)-2,6-dimethylimidazo[1,2-b]pyridazin-8-amine,
3-(1,3-dimethyl-1H-indazol-5-yl)-2,6-dimethyl-N-(thiophen-2-ylmethyl)imidazo[1,2-b]pyridazin-8-amine,
3-(1,3-dimethyl-1H-indazol-5-yl)-N-(4-methoxybenzyl)-2,6-dimethylimidazo[1,2-b]pyridazin-8-amine,
3-(1,3-dimethyl-1H-indazol-5-yl)-2,6-dimethyl-N-(4-(methylsulfonyl)benzyl)imidazo[1,2-b]pyridazin-8-amine,
3-(1,3-dimethyl-1H-indazol-5-yl)-2,5-dimethyl-N-(pyridin-4-ylmethyl)pyrazolo[1,5-a]pyrimidin-7-amine,
3-(1,3-dimethyl-1H-indazol-5-yl)-2,5-dimethyl-N-((6-methylpyridin-3-yl)methyl)pyrazolo[1,5-a]pyrimidin-7-amine,
3-(1,3-dimethyl-1H-indazol-5-yl)-N-(4-methoxybenzyl)-2,5-dimethylpyrazolo[1,5-a]pyrimidin-7-amine,
N-(4-chlorobenzyl)-3-(1,3-dimethyl-1H-indazol-5-yl)-2,5-dimethylpyrazolo[1,5-a]pyrimidin-7-amine,
3-(1,3-dimethyl-1H-indazol-5-yl)-2,5-dimethyl-N-(4-(methylsulfonyl)benzyl)pyrazolo[1,5-a]pyrimidin-7-amine,
4-(((3-(1,3-dimethyl-1H-indazol-5-yl)-2,5-dimethylpyrazolo[1,5-a]pyrimidin-7-yl)amino)methyl)benzenesulfonamide,
3-(1,3-dimethyl-1H-indazol-5-yl)-N-((6-methoxypyridin-3-yl)methyl)-2,5-dimethylpyrazolo[1,5-a]pyrimidin-7-amine,
3-(1,3-dimethyl-1H-indazol-5-yl)-2,5-dimethyl-N-(thiophen-2-ylmethyl)pyrazolo[1,5-a]pyrimidin-7-amine,
N-(4-(((3-(1,3-dimethyl-1H-indazol-5-yl)-2,5-dimethylpyrazolo[1,5-a]pyrimidin-7-yl)amino)methyl)phenyl)methanesulfonamide,
3-(1,3-dimethyl-1H-indazol-5-yl)-2,5-dimethyl-N-((6-trifluoromethylpyridin-3-yl)methyl)pyrazolo[1,5-a]pyrimidin-7-amine,
N-(benzyl)-3-(1,3-dimethyl-1H-indazol-5-yl)-2,5-dimethylpyrazolo[1,5-a]pyrimidin-7-amine,
N-(4-fluorobenzyl)-3-(1,3-dimethyl-1H-indazol-5-yl)-2,5-dimethylpyrazolo[1,5-a]pyrimidin-7-amine,
8-(1,3-dimethyl-1H-indazol-5-yl)-2,7-dimethyl-N-(pyridin-4-ylmethyl)pyrazolo[1,5-a][1,3,5]triazin-4-amine,
8-(1,3-dimethyl-1H-indazol-5-yl)-N-(4-methoxybenzyl)-2,7-dimethylpyrazolo[1,5-a][1,3,5]triazin-4-amine,
8-(1,3-dimethyl-1H-indazol-5-yl)-2,7-dimethyl-N-((6-methylpyridin-3-yl)methyl)pyrazolo[1, 5-a][1,3, 5]triazin-4-amine,
4-(((8-(1,3-dimethyl-1H-indazol-5-yl)-2,7-dimethylpyrazolo[1,5-a][1,3,5]triazin-4-yl)amino)methyl)benzenesulfonamide,
N-(4-chlorobenzyl)-8-(1,3-dimethyl-1H-indazol-5-yl)-2,7-dimethylpyrazolo[1,5-a][1,3,5]triazin-4-amine,
8-(1,3-dimethyl-1H-indazol-5-yl)-2,7-dimethyl-N-(4-(methylsulfonyl)benzyl)pyrazolo[1,5-a][1,3,5]triazin-4-amine,
N-(4-(((8-(1,3-dimethyl-1H-indazol-5-yl)-2,7-dimethylpyrazolo[1,5-a][1,3,5]triazin-4-yl)amino)methyl)phenyl)methanesulfonamide,
8-(1,3-dimethyl-1H-indazol-5-yl)-2,7-dimethyl-N-(thiophen-2-ylmethyl)pyrazolo[1,5-a][1,3,5]triazin-4-amine, and
8-(1,3-dimethyl-1H-indazol-5-yl)-N-((6-methoxypyridin-3-yl)methyl)-2,7-dimethylpyrazolo[1,5-a][1,3,5]triazin-4-amine.

11. The compound or pharmaceutically acceptable salt for use according to any one of claims 1 to 10, wherein the *Pneumoviridae* virus is an *Orthopneumovirus.*

12. The compound or pharmaceutically acceptable salt for use according to claim 11, wherein the *Orthopneumovirus* is a human respiratory syncytial virus.

13. The compound or pharmaceutically acceptable salt for use according to any one of claims 1 to 12, wherein the infection is a lower respiratory tract infection.

14. The compound 3-(3,4-dimethoxyphenyl)-2,6-dimethyl-N-(pyridin-4-ylmethyl)imidazo[1,2-b]pyridazin-8-amine, or pharmaceutically acceptable salt thereof.

15. A pharmaceutical composition comprising the compound of claim 14, or a pharmaceutically acceptable salt thereof, and optionally a pharmaceutically acceptable excipient.

16. The compound of claim 14, or a pharmaceutically acceptable salt thereof, for use in therapy.

## Patentansprüche

1. Verbindung der Formel (I) 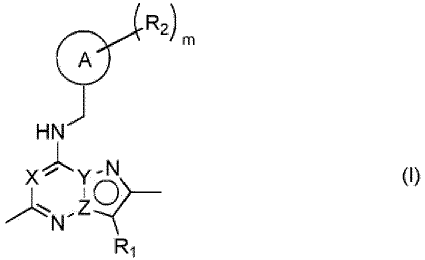 oder pharmazeutisch verträgliches Salz davon, wobei
X CH ist, Y C ist und Z N ist, oder
X CH ist, Y N ist und Z C ist, oder
X N ist, Y N ist und Z ist C;
R₁ 3,4-Dimethoxyphenyl oder 1,3-Dimethyl-1H-indazol-5-yl ist;
Ring A Phenyl oder 5- oder 6-gliedriges Heteroaryl ist;
m 0, 1, 2 oder 3 ist;
jedes R₂ unabhängig ausgewählt ist aus C1-C6-Alkyl, Halogen, R₃O, R₄R₅N, R₆R₇NS(O)₂, R₈R₉NC(O), R₁₀S(O)₂, R₁₁S(O)₂N(R₁₂), R₁₃C(O)O, R₁₄C(O)N(R₁₅), R₁₆OC(O), R₁₇C(O), R₁₈C(O)N(R₁₉)S(O)₂, R₂₀OC(O)N(R₂₁)S(O)2, CN und -O⁻; und zwei R₃, die an benachbarte Atome von Ring A gebunden sind, zusammen mit den Atomen, an die sie gebunden sind, einen 5- oder 6-gliedrigen heterocyclischen oder carbocyclischen Ring bilden können;
jedes R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀ und R₂₁ unabhängig ausgewählt ist aus H und C1-C6-Alkyl, und
jedes Alkyl gegebenenfalls durch ein oder mehrere F substituiert ist;
zur Verwendung bei der Behandlung einer Pneumoviridae-Virusinfektion.

2. Verbindung oder pharmazeutisch verträgliches Salz zur Verwendung gemäß Anspruch 1, wobei Ring A Phenyl oder 6-gliedriges Heteroaryl ist, das ein oder mehrere Stickstoffatome im Ring enthält.

3. Verbindung oder pharmazeutisch verträgliches Salz davon zur Verwendung gemäß Anspruch 1 oder 2, wobei Ring A Phenyl oder Pyridinyl ist.

4. Verbindung oder pharmazeutisch verträgliches Salz zur Verwendung gemäß einem der Ansprüche 1 bis 3, wobei Ring A 4-Pyridinyl ist.

5. Verbindung oder pharmazeutisch verträgliches Salz zur Verwendung gemäß einem der Ansprüche 1 bis 4, wobei X CH ist.

6. Verbindung oder pharmazeutisch verträgliches Salz zur Verwendung gemäß einem der Ansprüche 1 bis 5, wobei Y N ist und Z C ist.

7. Verbindung oder pharmazeutisch verträgliches Salz zur Verwendung gemäß einem der Ansprüche 1 bis 6, wobei R₁ 3,4-Dimethoxyphenyl ist.

8. Verbindung oder pharmazeutisch verträgliches Salz zur Verwendung gemäß einem der Ansprüche 1 bis 6, wobei R₁ 1,3-Dimethyl-1H-indazol-5-yl ist.

9. Verbindung oder pharmazeutisch verträgliches Salz zur Verwendung gemäß einem der Ansprüche 1 bis 8, wobei m 0 oder 1 ist.

10. Verbindung oder pharmazeutisch verträgliches Salz zur Verwendung gemäß Anspruch 1, wobei die Verbindung ausgewählt ist aus
3-(3,4-Dimethoxyphenyl)-2,6-dimethyl-N-(pyridin-4-ylmethyl)imidazo[1,2-b]pyridazin-8-amin,
3-(3,4-Dimethoxyphenyl)-2,6-dimethyl-N-((2-methylpyridin-4-yl)methyl)imidazo[1,2-b]pyridazin-8-amin,
3-(3,4-Dimethoxyphenyl)-N-((2-fluorpyridin-4-yl)methyl)-2,6-dimethylimidazo[1,2-b]pyridazin-8-amin,
3-(1,3-Dimethyl-1H-indazol-5-yl)-2,5-dimethyl-N-[(2-fluorpyridin-4-yl)methyl]pyrazolo[1,5-a]pyrimidin-7-amin,
3-(1,3-Dimethyl-1H-indazol-5-yl)-2,5-dimethyl-N-[(2-methylpyridin-4-yl)methyl]pyrazolo[1,5-a]pyrimidin-7-amin,
3-(1,3-Dimethyl-1H-indazol-5-yl)-2,5-dimethyl-N-[(2-ethylpyridin-4-yl)methyl]pyrazolo[1,5-a]pyrimidin-7-amin,
3-(1,3-Dimethyl-1H-indazol-5-yl)-2,5-dimethyl-N-[(2-methoxypyridin-4-yl)methyl]pyrazolo[1,5-a]pyrimidin-7-amin,
3-(1,3-Dimethyl-1H-indazol-5-yl)-2,5-dimethyl-N-[(2-cyanopyridin-4-yl)methyl]pyrazolo[1,5-a]pyrimidin-7-amin,
3-(1,3-Dimethyl-1H-indazol-5-yl)-2,5-dimethyl-N-[(2-trifluormethylpyridin-4-yl)methyl]pyrazolo[1,5-a ]pyrimidin-7-amin,
N-[(4-Chlorphenyl)methyl]-3-(3,4-dimethoxyphenyl)-2,5-dimethyl-pyrazolo[1,5-a]pyrimidin-7-amin,
3-(3,4-Dimethoxyphenyl)-N-[(4-methoxyphenyl)methyl]-2,5-dimethyl-pyrazolo[1,5-a]pyrimidin-7-amin,
3-(3,4-Dimethoxyphenyl)-2,5-dimethyl-N-(p-tolylmethyl)pyrazolo[1,5-a]pyrimidin-7-amin,
N-(1,3-Benzodioxol-5-ylmethyl)-3-(3,4-dimethoxyphenyl)-2,5-dimethyl-pyrazolo[1,5-a]pyrimidin-7-amin,
3-(3,4-Dimethoxyphenyl)-N-[(4-fluorphenyl)methyl]-2,5-dimethyl-pyrazolo[1,5-a]pyrimidin-7-amin,
3-(3,4-Dimethoxyphenyl)-2,5-dimethyl-N-(2-pyridylmethyl)pyrazolo[1,5-a]pyrimidin-7-amin,
N-Benzyl-3-(3,4-dimethoxyphenyl)-2,5-dimethyl-pyrazolo[1,5-a]pyrimidin-7-amin,
3-(3,4-Dimethoxyphenyl)-2,5-dimethyl-N-[[4-(trifluormethyl)phenyl]methyl]pyrazolo[1,5-a]pyrimidin-7-amin,
3-(3,4-Dimethoxyphenyl)-2,5-dimethyl-N-(4-pyridylmethyl)pyrazolo[1,5-a]pyrimidin-7-amin,
3-(3,4-Dimethoxyphenyl)-2,5-dimethyl-N-[[4-(trifluormethoxy)phenyl]methyl]pyrazolo[1,5-a]pyrimidin-7-amin,
N-[4-[[[3-(3,4-Dimethoxyphenyl)-2,5-dimethyl-pyrazolo[1,5-a]pyrimidin-7-yl]amino]methyl]phenyl]acetamid,
3-(3,4-Dimethoxyphenyl)-N-[(4-dimethylaminophenyl)methyl]-2,5-dimethyl-pyrazolo[1,5-a]pyrimidin-7-amin,
3-(3,4-Dimethoxyphenyl)-N-[(6-methoxy-3-pyridyl)methyl]-2,5-dimethyl-pyrazolo[1,5-a]pyrimidin-7-amin,
N-[4-[[[3-(3,4-Dimethoxyphenyl)-2,5-dimethyl-pyrazolo[1,5-a]pyrimidin-7-yl]amino]methyl]phenyl]methansulfonamid,
4-[[[3-(3,4-Dimethoxyphenyl)-2,5-dimethyl-pyrazolo[1,5-a]pyrimidin-7-yl]amino]methyl]phenol,
3-(3,4-Dimethoxyphenyl)-2,5-dimethyl-N-[(3-methylsulfonylphenyl)methyl] pyrazolo[1,5-a]pyrimidin-7-amin,
3-(3,4-Dimethoxyphenyl)-2,5-dimethyl-N-[(4-methylsulfonylphenyl)methyl]pyrazolo[1,5-a]pyrimidin-7-amin,
N-[(4-tert-Butylphenyl)methyl]-3-(3,4-dimethoxyphenyl)-2,5-dimethyl-pyrazolo[1,5-a]pyrimidin-7-amin,
3-(3,4-Dimethoxyphenyl)-2,5-dimethyl-N-[(2-methylpyrimidin-4-yl)methyl]pyrazolo[1,5-a]pyrimidin-7-amin,
3-(3,4-Dimethoxyphenyl)-2,5-dimethyl-N-[(6-methyl-3-pyridyl)methyl]pyrazolo[1,5-a]pyrimidin-7-amin,
1-[4-[[[3-(3,4-Dimethoxyphenyl)-2,5-dimethyl-pyrazolo[1,5-a]pyrimidin-7-yl]amino]methyl]phenyl]ethanon,
3-(3,4-Dimethoxyphenyl)-2,5-dimethyl-N-(1-naphthylmethyl)pyrazolo[1,5-a]pyrimidin-7-amin,
4-[[[3-(3,4-Dimethoxyphenyl)-2,5-dimethyl-pyrazolo[1,5-a]pyrimidin-7-yl]amino]methyl]benzolsulfonamid,
8-(3,4-Dimethoxyphenyl)-N-[(4-fluorphenyl)methyl]-2,7-dimethyl-pyrazolo[1,5-a][1,3,5 ]triazin-4-amin,
8-(3,4-Dimethoxyphenyl)-2,7-dimethyl-N-[[4-(trifluormethyl)phenyl]methyl]pyrazolo[1,5-a][1,3,5 ]triazin-4-amin,
8-(3,4-Dimethoxyphenyl)-2,7-dimethyl-N-(p-tolylmethyl)pyrazolo[1,5-a][1,3,5]triazin-4-amin,
8-(3,4-Dimethoxyphenyl)-N-[(4-isopropylphenyl)methyl]-2,7-dimethyl-pyrazolo[1,5-a][1,3,5]triazin-4-amin,
8-(3,4-Dimethoxyphenyl)-2,7-dimethyl-N-(2-pyridylmethyl)pyrazolo[1,5-a][1,3,5]triazin-4-amin,
8-(3,4- Dimethoxyphenyl)-2,7-dimethyl-N-(4-pyridylmethyl)pyrazolo[1,5-a][1,3,5]triazin-4-amin,
N-[(4-Chlorphenyl)methyl]-8-(3,4-dimethoxyphenyl)-2,7-dimethyl-pyrazolo[1,5-a][1,3,5]triazin-4-amin,
N-[4-[[[8-(3,4-Dimethoxyphenyl)-2,7-dimethyl-pyrazolo[1,5-a][1,3,5]triazin-4-yl]amino]methyl]phenyl]acetamid,
8-(3,4-Dimethoxyphenyl)-N-[(4-methoxyphenyl)methyl]-2,7-dimethyl-pyrazolo[1,5-a][1,3,5]triazin-4-amin,
8-(3,4-Dimethoxyphenyl)-2,7-dimethyl-N-[(3-methylsulfonylphenyl)methyl]pyrazolo[1,5-a][1,3,5]triazin-4-amin,
8-(3,4-Dimethoxyphenyl)-2,7-dimethyl-N-[(1-oxidopyridin-1-ium-4-yl)methyl]pyrazolo[1,5-a][1,3,5]triazin-4-amin,
8-(3,4-Dimethoxyphenyl)-N-[(6-methoxy-3-pyridyl)methyl]-2,7-dimethyl-pyrazolo[1,5-a][1,3,5]triazin-4-amin,
4-[[[8-(3,4-Dimethoxyphenyl)-2,7-dimethyl-pyrazolo[1,5-a][1,3,5]triazin-4-yl]amino]methyl]benzolsulfonamid,
N-(1,3-Benzodioxol-5-ylmethyl)-8-(3,4-dimethoxyphenyl)-2,7-dimethyl-pyrazolo[1,5-a][1,3,5]triazin-4-amin,
4-[[[8-(3,4-Dimethoxyphenyl)-2,7-dimethyl-pyrazolo[1,5-a][1,3,5]triazin-4-yl]amino]methyl]phenol,
N-[4-[[[8-(3,4-Dimethoxyphenyl)-2,7-dimethylpyrazolo[1,5-a][1,3,5]triazin-4-yl]amino]methyl]phenyl]methansulfonamid,
N-Benzyl-8-(3,4-dimethoxyphenyl)-2,7-dimethyl-pyrazolo[1,5-a][1,3,5]triazin-4-amin,
8-(3,4-Dimethoxyphenyl)-2,7-dimethyl-N-(2-thienylmethyl)pyrazolo[1,5-a][1,3,5]triazin-4-amin,
8-(3,4-Dimethoxyphenyl)-2,7-dimethyl-N-(3-pyridylmethyl)pyrazolo[1,5-a][1,3,5]triazin-4-amin,
8-(3,4-Dimethoxyphenyl)-2,7-dimethyl-N-[(5-methyl-2-furyl)methyl]pyrazolo[1,5 - a][1,3,5]triazin-4-amin,
Methyl-N-[4-[[[8-(3,4-dimethoxyphenyl)-2,7-dimethyl-pyrazolo[1,5-a][1,3,5]triazin-4-yl]amino]methyl]phenyl]sulfonylcarbamat,
N-[4-[[[8-(3,4-Dimethoxyphenyl)-2,7-dimethyl-pyrazolo[1,5- a][1,3,5]triazin-4-yl]amino]methyl]phenyl]sulfonylpropanamid,
N-Methyl-4-[[[8-(3,4-dimethoxyphenyl)-2,7-dimethyl-pyrazolo[1,5-a][1,3,5]triazin-4-yl]amino]methyl]benzolsulfonamid,
8-(3,4-Dimethoxyphenyl)-2,7-dimethyl-N-[(1-methyl-1H-pyrazol-3-yl)methyl]pyrazolo[1,5-a][1,3,5]triazin-4-amin,
8-(3,4-Dimethoxyphenyl)-2,7-dimethyl-N-[(1,3-oxazol-5-yl)methyl]pyrazolo[1,5-a][1,3,5]triazin-4-amin,
8-(3,4-Dimethoxyphenyl)-2,7-dimethyl-N-[(2-methylpyrimidin-5-yl)methyl]pyrazolo[1,5-a][1,3,5]triazin-4-amin,
8-(3,4-Dimethoxyphenyl)-2,7-dimethyl-N-[(pyridazin-4-yl)methyl]pyrazolo[1,5-a][1,3,5]triazin-4-amin,
8-(3,4-Dimethoxyphenyl)-2,7-dimethyl-N-[(6-methylpyridin-3-yl)methyl]pyrazolo[1,5-a][1,3,5]triazin-4-amin,
3-(1,3-Dimethyl-1H-indazol-5-yl)-2,6-dimethyl-N-(pyridin-4-ylmethyl)imidazo[1,2-b]pyridazin-8-amin,
3-(1,3-Dimethyl-1H-indazol-5-yl)-2,6-dimethyl-N-((6-methylpyridin-3-yl)methyl)imidazo[1,2-b]pyridazin-8-amin,
N-(4-Chlorbenzyl)-3-(1,3-dimethyl-1H-indazol-5-yl)-2,6-dimethylimidazo[1,2-b]pyridazin-8-amin,
3-(1,3-Dimethyl-1H-indazol-5-yl)-N-((6-methoxypyridin-3-yl)methyl)-2,6-dimethylimidazo[1,2-b]pyridazin-8-amin,
3-(1,3-Dimethyl-1H-indazol-5-yl)-2,6-dimethyl-N-(thiophen-2-ylmethyl)imidazo[1,2-b]pyridazin-8-amin,
3-(1,3-Dimethyl-1H-indazol-5-yl)-N-(4-methoxybenzyl)-2,6-dimethylimidazo[1,2-b]pyridazin-8-amin,
3-(1,3-Dimethyl-1H-indazol-5-yl)-2,6-dimethyl-N-(4-(methylsulfonyl)benzyl)imidazo[1,2-b]pyridazin-8-amin,
3-(1,3-Dimethyl-1H-indazol-5-yl)-2,5-dimethyl-N-(pyridin-4-ylmethyl)pyrazolo[1,5-a]pyrimidin-7-amin,
3-(1,3-Dimethyl-1H-indazol-5-yl)-2,5-dimethyl-N-((6-methylpyridin-3-yl) methyl)pyrazolo[1,5-a]pyrimidin-7-amin,
3-(1,3-Dimethyl-1H-indazol-5-yl)-N-(4-methoxybenzyl)-2,5-dimethylpyrazolo[1,5-a]pyrimidin-7-amin,
N-(4-Chlorbenzyl)-3-(1,3-dimethyl-1H-indazol-5-yl)-2,5-dimethylpyrazolo[1,5-a]pyrimidin-7-amin,
3-(1,3-Dimethyl-1H-indazol-5-yl)-2,5-dimethyl-N-(4-(methylsulfonyl)benzyl)pyrazolo[1,5-a]pyrimidin-7-amin,
4-(((3-(1,3-Dimethyl-1H-indazol-5-yl)-2,5-dimethylpyrazolo[1,5-a]pyrimidin-7-yl)amino)methyl)benzolsulfonamid,
3-(1,3-Dimethyl-1H-indazol-5-yl)-N-((6-methoxypyridin-3-yl)methyl)-2,5-dimethylpyrazolo[1,5-a]pyrimidin-7-amin,
3-(1,3-Dimethyl-1H-indazol-5-yl)-2,5-dimethyl-N-(thiophen-2-ylmethyl)pyrazolo[1,5-a]pyrimidin-7-amin,
N-(4-(((3-(1,3-Dimethyl-1H-indazol-5-yl)-2,5-dimethylpyrazolo[1,5-a]pyrimidin-7-yl)amino)methyl)phenyl)methansulfonamid,
3-(1,3-Dimethyl-1H-indazol-5-yl)-2,5-dimethyl-N-((6-trifluormethylpyridin-3-yl)methyl)pyrazolo[1,5-a]pyrimidin-7-amin,
N-(Benzyl)-3-(1,3-dimethyl-1H-indazol-5-yl)-2,5-dimethylpyrazolo[1,5-a]pyrimidin-7-amin,
N-(4-Fluorbenzyl)-3-(1,3-dimethyl-1H-indazol-5-yl)-2,5-dimethylpyrazolo[1,5-a]pyrimidin-7-amin,
8-(1,3-Dimethyl-1H-indazol-5-yl)-2,7-dimethyl-N-(pyridin-4-ylmethyl)pyrazolo[1,5-a][1,3,5]triazin-4-amin,
8-(1,3-Dimethyl-1H-Indazol-5-yl)-N-(4-methoxybenzyl)-2,7-dimethylpyrazolo[1,5-a][1,3,5]triazin-4-amin,
8-(1,3-Dimethyl)-1H-indazol-5-yl)-2,7-dimethyl-N-((6-methylpyridin-3-yl)methyl)pyrazolo[1,5-a][1,3,5]triazin-4-amin,
4-(((8-(1,3-Dimethyl-1H-indazol-5-yl)-2,7-dimethylpyrazolo[1,5-a][1,3,5]triazin-4-yl)amino)methyl)benzolsulfonamid,
N-(4-Chlorbenzyl)-8-(1,3-dimethyl-1H-indazol-5-yl)-2,7-dimethylpyrazolo[1,5-a][1,3,5] triazin-4-amin,
8-(1,3-Dimethyl-1H-indazol-5-yl)-2,7-dimethyl-N-(4-(methylsulfonyl)benzyl)pyrazolo[1,5-a][1,3,5]triazin-4-amin,
N-(4-(((8-(1,3-Dimethyl-1H-indazol-5-yl)-2,7-dimethylpyrazolo[1,5-a][1,3,5]triazin-4-yl)amino)methyl)phenyl)methansulfonamid,
8-(1,3-Dimethyl-1H-indazol-5-yl)-2,7-dimethyl-N-(thiophen-2-ylmethyl)pyrazolo[1,5-a][1,3,5]triazin-4-amin und
8-(1,3-Dimethyl-1H-indazol-5-yl)-N-((6-methoxypyridin-3-yl)methyl)-2,7-dimethylpyrazolo[1,5-a][1,3,5]triazin-4-amin.

11. Verbindung oder pharmazeutisch verträgliches Salz zur Verwendung gemäß einem der Ansprüche 1 bis 10, wobei es sich bei dem *Pneumoviridae*-Virus um ein *Orthopneumovirus* handelt.

12. Verbindung oder pharmazeutisch verträgliches Salz zur Verwendung gemäß Anspruch 11, wobei es sich bei dem *Orthopneumovirus* ein humanes Respiratory-Syncytial-Virus handelt.

13. Verbindung oder pharmazeutisch verträgliches Salz zur Verwendung gemäß einem der Ansprüche 1 bis 12, wobei es sich bei der Infektion um eine Infektion der unteren Atemwege handelt.

14. Die Verbindung 3-(3,4-Dimethoxyphenyl)-2,6-dimethyl-N-(pyridin-4-ylmethyl)imidazo[1,2-b]pyridazin-8-amin oder ein pharmazeutisch verträgliches Salz davon.

15. Pharmazeutische Zusammensetzung, umfassend die Verbindung nach Anspruch 14 oder ein pharmazeutisch verträgliches Salz davon und gegebenenfalls einen pharmazeutisch verträglichen Hilfsstoff.

16. Verbindung nach Anspruch 14 oder pharmazeutisch verträgliches Salz davon zur Verwendung in der Therapie.

## Revendications

1. Composé de formule (I) 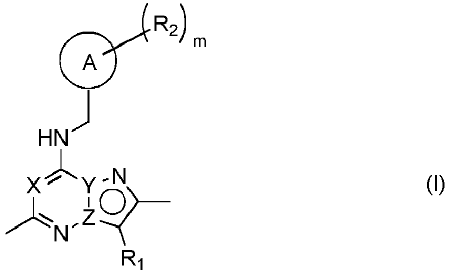 ou un sel pharmaceutiquement acceptable de celui-ci, dans lequel
X est CH, Y est C, et Z est N, ou
X est CH, Y est N, et Z est C, ou
X est N, Y est N, et Z est C ;
R₁ est un 3,4-diméthoxyphényle ou un 1,3-diméthyl-1H-indazol-5-yle ;
le cycle A est un phényl, ou un hétéroaryle à 5 ou 6 chaînons ;
m est 0, 1, 2 ou 3 ;
chaque R₂ est indépendamment sélectionné parmi un alkyle en C1-C6, un halogène, R₃O, R₄R₅N, R₆R₇NS(O)₂, R₈R₉NC(O), R₁₀S(O)₂, R₁₁S(O)₂N(R₁₂), R₁₃C(O)O, R₁₄C(O)N(R₁₅), R₁₆OC(O), R₁₇C(O), R₁₈C(O)N(R₁₉)S(O)2, R₂₀OC(O)N(R₂₁)S(O)₂, CN et - O⁻ ; et deux R₃, liés à des atomes adjacents du cycle A, conjointement aux atomes auxquels ils sont liés peuvent former un cycle hétérocyclique ou carbocyclique à 5 ou 6 chaînons ;
chaque R₃, R₄, R₅, R₆, R₇, R₈, R₉, R₁₀, R₁₁, R₁₂, R₁₃, R₁₄, R₁₅, R₁₆, R₁₇, R₁₈, R₁₉, R₂₀, et R₂₁ est indépendamment sélectionné parmi H et un alkyle en C1-C6 ; et
tout alkyle est facultativement substitué par un ou plusieurs F ;
pour son utilisation dans le traitement d'une infection virale à *Pneumoviridae.*

2. Composé ou sel pharmaceutiquement acceptable pour son utilisation selon la revendication 1, dans lequel le cycle A est un phényle ou un hétéroaryle à 6 chaînons contenant un ou plusieurs atomes d'azote dans le cycle .

3. Composé ou sel pharmaceutiquement acceptable de celui-ci pour son utilisation selon la revendication 1 ou 2, dans lequel le cycle A est un phényle ou un pyridinyle.

4. Composé ou sel pharmaceutiquement acceptable pour son utilisation selon l'une quelconque des revendications 1 à 3, dans lequel le cycle A est un 4-pyridinyle.

5. Composé ou sel pharmaceutiquement acceptable pour son utilisation selon l'une quelconque des revendications 1 à 4, dans lequel X est CH .

6. Composé ou sel pharmaceutiquement acceptable pour son utilisation selon l'une quelconque des revendications 1 à 5, dans lequel Y est N et Z est C.

7. Composé ou sel pharmaceutiquement acceptable pour son utilisation selon l'une quelconque des revendications 1 à 6, dans lequel R₁ est un 3,4-diméthoxyphényle.

8. Composé ou sel pharmaceutiquement acceptable pour son utilisation selon l'une quelconque des revendications 1 à 6, dans lequel R₁ est un 1,3-diméthyl-1H-indazol-5-yle.

9. Composé ou sel pharmaceutiquement acceptable pour son utilisation selon l'une quelconque des revendications 1 à 8, dans lequel m est 0 ou 1.

10. Composé ou sel pharmaceutiquement acceptable pour son utilisation selon la revendication 1, dans lequel le composé est sélectionné parmi les
3-(3,4-diméthoxyphényl)-2,6-diméthyl-N-(pyridin-4-ylméthyl)imidazo[1,2-b]pyridazin-8-amine,
3-(3,4-diméthoxyphényl)-2,6-diméthyl-N-((2-méthylpyridin-4-yl)méthyl)imidazo[1,2-b]pyridazin-8-amine,
3-(3,4-diméthoxyphényl)-N-((2-fluoropyridin-4-yl)méthyl)-2,6-diméthylimidazo[1,2-b]pyridazin-8-amine,
3-(1,3-diméthyl-1H-indazol-5-yl)-2,5-diméthyl-N-[(2-fluoropyridin-4-yl)méthyl]pyrazolo[1,5-a]pyrimidin-7-amine,
3-(1,3-diméthyl-1H-indazol-5-yl)-2,5-diméthyl-N-[(2-méthylpyridin-4-yl)méthyl]pyrazolo[1,5-a]pyrimidin-7-amine,
3-(1,3-diméthyl-1H-indazol-5-yl)-2,5-diméthyl-N-[(2-éthylpyridin-4-yl)méthyl]pyrazolo[1,5-a]pyrimidin-7-amine,
3-(1,3-diméthyl-1H-indazol-5-yl)-2,5-diméthyl-N-[(2-méthoxypyridin-4-yl)méthyl]pyrazolo[1,5-a]pyrimidin-7-amine,
3-(1,3-diméthyl-1H-indazol-5-yl)-2,5-diméthyl-N-[(2-cyanopyridin-4-yl)méthyl]pyrazolo[1,5-a]pyrimidin-7-amine,
3-(1,3-diméthyl-1H-indazol-5-yl)-2,5-diméthyl-N-[(2-trifluorométhylpyridin-4-yl)méthyl]pyrazolo[1,5-a]pyrimidin-7-amine,
N-[(4-chlorophényl)méthyl]-3-(3,4-diméthoxyphényl)-2,5-diméthyl-pyrazolo[1,5-a]pyrimidin-7-amine,
3-(3,4-diméthoxyphényl)-N-[(4-méthoxyphényl)méthyl]-2,5-diméthyl-pyrazolo[1,5-a]pyrimidin-7-amine,
3-(3,4-diméthoxyphényl)-2,5-diméthyl-N-(p-tolylméthyl)pyrazolo[1,5-a]pyrimidin-7-amine,
N-(1,3-benzodioxol-5-ylméthyl)-3-(3,4-diméthoxyphényl)-2,5-diméthyl-pyrazolo[1,5-a]pyrimidin-7-amine,
3-(3,4-diméthoxyphényl)-N-[(4-fluorophényl)méthyl]-2,5-diméthyl-pyrazolo[1,5-a]pyrimidin-7-amine,
3-(3,4-diméthoxyphényl)-2,5-diméthyl-N-(2-pyridylméthyl)pyrazolo[1,5-a]pyrimidin-7-amine,
N-benzyl-3-(3,4-diméthoxyphényl)-2,5-diméthyl-pyrazolo[1,5-a]pyrimidin-7-amine,
3-(3,4-diméthoxyphényl)-2,5-diméthyl-N-[[4-(trifluorométhyl)phényl]méthyl]pyrazolo[1,5-a]pyrimidin-7-amine,
3-(3,4-diméthoxyphényl)-2,5-diméthyl-N-(4-pyridylméthyl)pyrazolo[1,5-a]pyrimidin-7-amine,
3-(3,4-diméthoxyphényl)-2,5-diméthyl-N-[[4-(trifluorométhoxy)phényl]méthyl]pyrazolo[1,5-a]pyrimidin-7-amine,
N-[4-[[[3-(3,4-diméthoxyphényl)-2,5-diméthyl-pyrazolo[1,5-a]pyrimidin-7-yl]amino]méthyl] phényl]acétamide,
3-(3,4-diméthoxyphényl)-N-[(4-diméthylaminophényl)méthyl]-2,5-diméthyl-pyrazolo[1,5-a]pyrimidin-7-amine,
3-(3,4-diméthoxyphényl)-N-[(6-méthoxy-3-pyridyl)méthyl]-2,5-diméthyl-pyrazolo[1,5-a]pyrimidin-7-amine,
N-[4-[[[3-(3,4-diméthoxyphényl)-2,5-diméthyl-pyrazolo[1,5-a]pyrimidin-7-yl]amino]méthyl]phényl]méthanesulfonamide,
4-[[[3-(3,4-diméthoxyphényl)-2,5-diméthyl-pyrazolo[1,5-a]pyrimidin-7-yl]amino]méthyl]phénol,
3-(3,4-diméthoxyphényl)-2,5-diméthyl-N-[(3-méthylsulfonylphényl)méthyl]pyrazolo[1,5-a]pyrimidin-7-amine,
3-(3,4-diméthoxyphényl)-2,5-diméthyl-N-[(4-méthylsulfonylphényl)méthyl]pyrazolo[1,5-a]pyrimidin-7-amine,
N-[(4-tert-butylphényl)méthyl]-3-(3,4-diméthoxyphényl)-2,5-diméthyl-pyrazolo[1,5-a]pyrimidin-7-amine,
3-(3,4-diméthoxyphényl)-2,5-diméthyl-N-[(2-méthylpyrimidin-4-yl)méthyl]pyrazolo[1,5-a]pyrimidin-7-amine,
3-(3,4-diméthoxyphényl)-2,5-diméthyl-N-[(6-méthyl-3-pyridyl)méthyl]pyrazolo[1,5-a]pyrimidin-7-amine,
1-[4-[[[3-(3,4-diméthoxyphényl)-2,5-diméthyl-pyrazolo[1,5-a]pyrimidin-7-yl]amino]méthyl]phényl]éthanone,
3-(3,4-diméthoxyphényl)-2,5-diméthyl-N-(1-naphtylméthyl)pyrazolo[1,5-a]pyrimidin-7-amine,
4-[[[3-(3,4-diméthoxyphényl)-2,5-diméthyl-pyrazolo[1,5-a]pyrimidin-7-yl]amino]méthyl]benzènesulfonamide,
8-(3,4-diméthoxyphényl)-N-[(4-fluorophényl)méthyl]-2,7-diméthyl-pyrazolo[1,5-a][1,3,5]triazin-4-amine,
8-(3,4-diméthoxyphényl)-2,7-diméthyl-N-[[4-(trifluorométhyl)phényl]méthyl]pyrazolo[1,5-a][1,3,5]triazin-4-amine,
8-(3,4-diméthoxyphényl)-2,7-diméthyl-N-(p-tolylméthyl)pyrazolo[1,5-a][1,3,5]triazin-4-amine,
8-(3,4-diméthoxyphényl)-N-[(4-isopropylphényl)méthyl]-2,7-diméthyl-pyrazolo[1,5-a][1,3,5]triazin-4-amine,
8-(3,4-diméthoxyphényl)-2,7-diméthyl-N-(2-pyridylméthyl)pyrazolo[1,5-a][1,3,5]triazin-4-amine,
8-(3,4-diméthoxyphényl)-2,7-diméthyl-N-(4-pyridylméthyl)pyrazolo[1,5-a][1,3,5]triazin-4-amine,
N-[(4-chlorophényl)méthyl]-8-(3,4-diméthoxyphényl)-2,7-diméthyl-pyrazolo[1,5-a][1,3,5]triazin-4-amine,
N-[4-[[[8-(3,4-diméthoxyphényl)-2,7-diméthyl-pyrazolo[1,5-a][1,3,5]triazin-4-yl]amino]méthyl]phényl]acétamide,
8-(3,4-diméthoxyphényl)-N-[(4-méthoxyphényl)méthyl]-2,7-diméthyl-pyrazolo[1,5-a][1,3,5]triazin-4-amine,
8-(3,4-diméthoxyphényl)-2,7-diméthyl-N-[(3-méthylsulfonylphényl)méthyl]pyrazolo[1,5-a][1,3,5]triazin-4-amine,
8-(3,4-diméthoxyphényl)-2,7-diméthyl-N-[(l-oxidopyridin-1-ium-4-yl)méthyl]pyrazolo[1,5-a][1,3,5]triazin-4-amine,
8-(3,4-diméthoxyphényl)-N-[(6-méthoxy-3-pyridyl)méthyl]-2,7-diméthyl-pyrazolo[1,5-a][1,3,5]triazin-4-amine,
4-[[[8-(3,4-diméthoxyphényl)-2,7-diméthyl-pyrazolo[1,5-a][1,3,5]triazin-4-yl]amino]méthyl]benzènesulfonamide,
N-(1,3-benzodioxol-5-ylméthyl)-8-(3,4-diméthoxyphényl)-2,7-diméthyl-pyrazolo[1,5-a][1,3,5]triazin-4-amine,
4-[[[8-(3,4-diméthoxyphényl)-2,7-diméthyl-pyrazolo[1,5-a][1,3,5]triazin-4-yl]amino]méthyl] phénol,
N-[4-[[[8-(3,4-diméthoxyphényl)-2,7-diméthyl-pyrazolo[1,5-a][1,3,5]triazin-4-yl]amino]méthyl]phényl]méthanesulfonamide,
N-benzyl-8-(3,4-diméthoxyphényl)-2,7-diméthyl-pyrazolo[1,5-a][1,3,5]triazin-4-amine,
8-(3,4-diméthoxyphényl)-2,7-diméthyl-N-(2-thiénylméthyl)pyrazolo[1,5-a][1,3,5]triazin-4-amine,
8-(3,4-diméthoxyphényl)-2,7-diméthyl-N-(3-pyridylméthyl)pyrazolo[1,5-a][1,3,5]triazin-4-amine,
8-(3,4-diméthoxyphényl)-2,7-diméthyl-N-[(5-méthyl-2-furyl)méthyl]pyrazolo[1,5-a][1,3,5]triazin-4-amine,
N-[4-[[[8-(3,4-diméthoxyphényl)-2,7-diméthyl-pyrazolo[1,5-a][1,3,5]triazin-4-yl]amino]méthyl]phényl]sulfonylcarbamate de méthyle,
N-[4-[[[8-(3,4-diméthoxyphényl)-2,7-diméthyl-pyrazolo[1,5-a][1,3,5]triazin-4-yl]amino]méthyl]phényl]sulfonylpropanamide,
N-méthyl-4-[[[8-(3,4-diméthoxyphényl)-2,7-diméthyl-pyrazolo[1,5-a][1,3,5]triazin-4-yl]amino]méthyl]benzènesulfonamide,
8-(3,4-diméthoxyphényl)-2,7-diméthyl-N-[(1-méthyl-1H-pyrazol-3-yl)méthyl]pyrazolo[1,5-a][1,3,5]triazin-4-amine,
8-(3,4-diméthoxyphényl)-2,7-diméthyl-N-[(1,3-oxazol-5-yl)méthyl]pyrazolo[1,5-a][1,3,5]triazin-4-amine,
8-(3,4-diméthoxyphényl)-2,7-diméthyl-N-[(2-méthylpyrimidin-5-yl)méthyl]pyrazolo[1,5-a][1,3,5]triazin-4-amine,
8-(3,4-diméthoxyphényl)-2,7-diméthyl-N-[(pyridazin-4-yl)méthyl]pyrazolo[1,5-a][1,3,5]triazin-4-amine,
8-(3,4-diméthoxyphényl)-2,7-diméthyl-N-[(6-méthylpyridin-3-yl)méthyl]pyrazolo[1,5-a][1,3,5]triazin-4-amine,
3-(1,3-diméthyl-1H-indazol-5-yl)-2,6-diméthyl-N-(pyridin-4-ylméthyl)imidazo[1,2-b]pyridazin-8-amine,
3-(1,3-diméthyl-1H-indazol-5-yl)-2,6-diméthyl-N-((6-méthylpyridin-3-yl)méthyl)imidazo[1,2-b]pyridazin-8-amine,
N-(4-chlorobenzyl)-3-(1,3-diméthyl-1h-indazol-5-yl)-2,6-diméthylimidazo[1,2-b]pyridazin-
8-amine,
3-(1,3-diméthyl-1H-indazol-5-yl)-N-((6-méthoxypyri din-3-yl)méthyl)-2,6-diméthylimidazo[1,2-b]pyridazin-8-amine,
3-(1,3-diméthyl-1h-indazol-5-yl)-2,6-diméthyl-N-(thiophén-2-ylméthyl)imidazo[1,2-b]pyridazin-8-amine,
3-(1,3-diméthyl-1h-indazol-5-yl)-N-(4-méthoxybenzyl)-2,6-diméthylimidazo[1,2-b]pyridazin-8-amine,
3-(1,3-diméthyl-1H-indazol-5-yl)-2,6-diméthyl-N-(4-(méthylsulfonyl)benzyl)imidazo[1,2-b]pyridazin-8-amine,
3-(1,3-diméthyl-1H-indazol-5-yl)-2,5-diméthyl-N-(pyridin-4-ylméthyl)pyrazolo[1,5-a]pyrimidin-7-amine,
3-(1,3-diméthyl-1H-indazol-5-yl)-2,5-diméthyl-N-((6-méthylpyridin-3-yl)méthyl)pyrazolo[1,5-a]pyrimidin-7-amine,
3-(1,3-diméthyl-1h-indazol-5-yl)-N-(4-méthoxybenzyl)-2,5-diméthylpyrazolo[1,5-a]pyrimidin-7-amine,
N-(4-chlorobenzyl)-3-(1,3-diméthyl-1h-indazol-5-yl)-2,5-diméthylpyrazolo[1,5-a]pyrimidin-7-amine,
3-(1,3-diméthyl-1H-indazol-5-yl)-2,5-diméthyl-N-(4-(méthylsulfonyl)benzyl)pyrazolo[1,5-a]pyrimidin-7-amine,
4-(((3-(1,3-diméthyl-1H-indazol-5-yl)-2,5-diméthylpyrazolo[1,5-a]pyrimidin-7-yl)amino)méthyl)benzènesulfonamide,
3-(1,3-diméthyl-1H-indazol-5-yl)-N-((6-méthoxypyridin-3-yl)méthyl)-2,5-diméthylpyrazolo[1,5-a]pyrimidin-7-amine,
3-(1,3-diméthyl-1H-indazol-5-yl)-2,5-diméthyl-N-(thiophén-2-ylméthyl)pyrazolo[1,5-a]pyrimidin-7-amine,
N-(4-(((3-(1,3-diméthyl-1H-indazol-5-yl)-2,5-diméthylpyrazolo[1,5-a]pyrimidin-7-yl)amino)méthyl)phényl)méthanesulfonamide,
3-(1,3-diméthyl-1H-indazol-5-yl)-2,5-diméthyl-N-((6-trifluorométhylpyridin-3-yl)méthyl)pyrazolo[1,5-a]pyrimidin-7-amine,
N-(benzyl)-3-(1,3-diméthyl-1h-indazol-5-yl)-2,5-diméthylpyrazolo[1,5-a]pyrimidin-7-amine,
N-(4-fluorobenzyl)-3-(1,3-diméthyl-1H-indazol-5-yl)-2,5-diméthylpyrazolo[1,5-a]pyrimidin-7-amine,
8-(1,3-diméthyl-1H-indazol-5-yl)-2,7-diméthyl-N-(pyridin-4-ylméthyl)pyrazolo[1,5-a][1,3,5]triazin-4-amine,
8-(1,3-diméthyl-1H-indazol-5-yl)-N-(4-méthoxybenzyl)-2,7-diméthylpyrazolo[1,5-a][1,3,5]triazin-4-amine,
8-(1,3-diméthyl-1H-indazol-5-yl)-2,7-diméthyl-N-((6-méthylpyridin-3-yl)méthyl)pyrazolo[1,5-a][1,3,5]triazin-4-amine,
4-(((8-(1,3-diméthyl-1H-indazol-5-yl)-2,7-diméthylpyrazolo[1,5-a][1,3,5]triazin-4-yl)amino)méthyl)benzènesulfonamide,
N-(4-chlorobenzyl)-8-(1,3-diméthyl-1H-indazol-5-yl)-2,7-diméthylpyrazolo[1,5-a][1,3,5]triazin-4-amine,
8-(1,3-diméthyl-1H-indazol-5-yl)-2,7-diméthyl-N-(4-(méthylsulfonyl)benzyl)pyrazolo[1,5-a][1,3,5]triazin-4-amine,
N-(4-(((8-(1,3-diméthyl-1H-indazol-5-yl)-2,7-diméthylpyrazolo[1,5-a][1,3,5]triazin-4-yl)amino)méthyl)phényl)méthanesulfonamide,
8-(1,3-diméthyl-1H-indazol-5-yl)-2,7-diméthyl-N-(thiophén-2-ylméthyl)pyrazolo[1,5-a][1,3,5]triazin-4-amine, et
8-(1,3-diméthyl-1H-indazol-5-yl)-N-((6-méthoxypyridin-3-yl)méthyl)-2,7-diméthylpyrazolo[1,5-a][1,3,5]triazin-4-amine.

11. Composé ou sel pharmaceutiquement acceptable pour son utilisation selon l'une quelconque des revendications 1 à 10, dans lequel le virus *Pneumoviridae* est un *Orthopneumovirus.*

12. Composé ou sel pharmaceutiquement acceptable pour son utilisation selon la revendication 11, dans lequel l'*Orthopneumovirus* est un virus respiratoire syncytial humain.

13. Composé ou sel pharmaceutiquement acceptable pour son utilisation selon l'une quelconque des revendications 1 à 12, dans lequel l'infection est une infection de voies respiratoires inférieures.

14. Composé 3-(3,4-diméthoxyphényl)-2,6-diméthyl-N-(pyridin-4-ylméthyl)imidazo[1,2-b]pyridazin-8-amine, ou sel pharmaceutiquement acceptable de celui-ci.

15. Composition pharmaceutique comprenant le composé selon la revendication 14, ou un sel pharmaceutiquement acceptable de celui-ci, et facultativement un excipient pharmaceutiquement acceptable .

16. Composé selon la revendication 14, ou sel pharmaceutiquement acceptable de celui-ci, pour son utilisation en thérapie.
